Europäisches Patentamt

European Patent Office

Office européen des brevets

(11) Publication number: **0 167 238**
**B1**

# EUROPEAN PATENT SPECIFICATION

(45) Date of publication of patent specification: **01.06.88**

(51) Int. Cl.⁴: **C 12 Q 1/68**

(21) Application number: **85303175.5**

(22) Date of filing: **03.05.85**

(54) **Displacement polynucleotide assay method and polynucleotide complex reagent therefor.**

(30) Priority: **07.05.84 US 607885**
**20.12.84 US 684308**

(43) Date of publication of application:
**08.01.86 Bulletin 86/02**

(45) Publication of the grant of the patent:
**01.06.88 Bulletin 88/22**

(84) Designated Contracting States:
**AT BE CH DE FR GB IT LI NL SE**

(56) References cited:
**EP-A-0 070 685**
**EP-A-0 124 221**
**EP-A-0 130 515**
**US-A-4 302 204**
**US-A-4 358 535**
**US-A-4 395 486**

(73) Proprietor: **ALLIED CORPORATION**
**Columbia Road and Park Avenue P.O. Box 2245R**
**(Law Dept.)**
**Morristown New Jersey 07960 (US)**
(73) Proprietor: **GENETICS INSTITUTE, INC.**
**87 Cambridgepark Drive**
**Cambridge Massachusetts 02140 (US)**

(72) Inventor: **Collins, Mary**
**27 Euclid Avenue**
**Natick Massachusetts 01760 (US)**
Inventor: **Fritsch, Edward Francis**
**115 North Branch Road**
**Concord Massachusetts 01742 (US)**
Inventor: **Williams, Jon Ira**
**123 North Mountain Avenue**
**Montclair New Jersey 07042 (US)**
Inventor: **Brewen, Joseph Grant**
**17 Concord Lane**
**Convent Station New Jersey 07961 (US)**
Inventor: **Diamond, Steven Elliot**
**30 Park Lane**
**Springfield New Jersey 07081 (US)**
Inventor: **Ellwood, Marian Sue**
**16 Russell Place**
**Summit New Jersey 07901 (US)**

Courier Press, Leamington Spa, England.

**0 167 238**

(74) Representative: **Myerscough, Philip Boyd et al**
**J.A.Kemp & Co. 14, South Square Gray's Inn**
**London, WC1R 5EU (GB)**

**Description**

Background of the invention

The present invention relates to a diagnostic assay method for detecting the presence of a target nucleotide sequence (either DNA or RNA) in a biological sample, and to a polynucleotide reagent complex therefor.

Conventional methods for detecting the presence of a particular polynucleotide in a biological sample typically involve immobilization of nucleic acid of the sample on a surface as the initial step. Once the sample is immobilized, a probe polynucleotide strand, tagged usually with a detectable label such as radioactive phosphorus atoms, is incubated with the immobilized sample so as to bind to the immobilized sample by purine/pyrimidine base sequence-specific complementary base pairing when the immobilized sample contains the target nucleotide sequence. After washing off the labelled probe which has not so hybridized, the presence or absence of label on the support is then determined. Techniques for this determination include exposure of a photographic film, liquid scintillation counting, and fluorescence microscopy.

Ward and coworkers (see EP—A—63,879) have described a variation of this technique in which, rather than tagging the probe directly with a detectable label, the probe is tagged with a nonisotopic substituent such as biotin on certain nucleotides. In such case, after the unhybridized probe is washed off, the support is contacted with a reagent such as avidin linked to an enzyme. The avidin-enzyme complex binds selectively to biotin because of the high avidin-biotin binding affinity, so as to affix enzyme selectively where the target nucleotide sequence has been immobilized on the substrate. Thereafter, a substrate for the enzyme is added and products of the enzymatic reaction are detected, yielding an amplified signal functionally dependent upon the initial concentration of target nucleotide sequence on the substrate. See also EPA 97,373 of Enzo Biochem, Inc. (January 4, 1984).

A variation in the above nonisotopic system has also been described in another European patent application of Standard Oil of Illinois (EPA 70,687 (1983)) in which, in one form (see pages 8—10 thereof), two probes specific for the target nucleotide sequence are employed. The first probe, which can hybridize to a first portion of the target nucleotide sequence, is affixed to a solid support such that, upon incubation of the solid support with a sample of the biological material, target nucleotide sequences in the sample will bind to the support selectively via this first immobilized probe. Thereafter, the second probe, which can hybridize selectively to a second and distinct portion of the target nucleotide sequence, is used to contact the support. Again, if the target nucleotide sequence had been present in the biological sample, the second probe will now bind selectively to that nucleotide sequence; and a combination structure (or sandwich) will be created linking the second probe to the support via the first probe and the target nucleotide sequence. The reference discloses labelling this second probe with a moiety directly or indirectly generating or absorbing specific wavelengths of light (e.g., a fluorescent label, a phosphorescent label or a chemiluminescent label). By separating the support from unbound solution constituents at each stage, the presence of label in the phase with support after the third separation will be a function of the presence and concentration of the target nucleotide sequence in the sample. See also WO 83/01459 of Orion-Yhtma Oy (April 29, 1983).

A different diagnostic method for a specific target nucleotide involving digestion of double-stranded sample nucleic acid in solution with a restriction enzyme, followed by detection of specifically sized fragments on filter paper, is disclosed in U.S. Patent 4,395,486 to Wilson et al. In that disclosure, the presence of the single base substitution causative of sickle cell anemia abolishes a specific site for restriction enzyme cleavage, and thereafter two specifically sized small fragments which are usually detected are then detected in reduced amounts (for sickle cell trait) or cease to be detected (for sickle cell anemia).

While the above procedures will detect the presence of nucleotide sequences in biological samples in many cases, they each have the disadvantage of either multiple steps or steps with necessarily long incubation periods that make them impracticable for easy use in a clinical laboratory. Furthermore, many of these processes suffer from a limited selectivity or sensitivity with regard to interfering polynucleotide sequences or the detection of low levels of target nucleotide sequence reliably against the background signal. In particular, nonspecific binding of the labelled probe represents a source of substantial background signal in each process.

Apart from the analysis of biological samples for target nucleotide sequences, various aspects of the physical chemistry of hybridization (formation of double-stranded helices between complementary polynucleotide sequences) have been studied. These studies have included examination of the phenomena of strand migration and displacement in nucleic acid, both *in vivo* and *in vitro*. By referring to such studies, however, we do not admit that the phenomena of strand migration and displacement have any obvious applicability to diagnosis and detection. C. Green and C. Tibbetts, *Nucleic Acids Research* vol. 9, No. 8, pp. 1905—18 (1981), have described the formation of a complex (hybrid) of a 6.1 kb (6100 base long) single-stranded DNA polynucleotide hybridized near its middle (the interval 1.7—3.3 kb) by an end-labelled complementary DNA polynucleotide of 1.6 kb length. Addition to this complex, in solution, of the 6.1 kb complementary strand caused rapid displacement of the labelled polynucleotide (see Fig. 2 on page 1910 of this reference), monitored by taking aliquots of the reaction mixture, separating then by gel chromatography and analyzing then by autoradiography. The displaced 1.6 kb polynucleotide increased steadily from under 10% to over 90% of the radioactive signal in a period of more than 85 minutes (depending upon concentration) with the 1.6/6.1 kb hybrid accounting for the bulk of the remaining radioactivity. The presumed partially displaced intermediate, which would have a total mass equivalent to 13.8 kb of DNA (both long strands and a partially displaced short strand) was apparently not detected. The authors concluded that the initial hybridization of the two 6.1 kb polynucleotides, forming a branched species, was the rate-limiting step; and that displacement along the 1.6 kb paired region of a labelled polynucleotide was very rapid, consistent with a calculated average lifetime of the branched (13.8 kb mass equivalent) species of 0.8 minutes. They indicate the possibility of both single-branched or doubly nucleated (D-looped) intermediate species (illustrated on page 1912 of the reference). In order to better study the phenomenon of branch migration, they attempted to slow the displacement process, by using drugs which might retard the migration phenomenon and/or by using complexes with more than 1.6 kb of hybrid base pairing (see pages 1913—1914 of the reference). It should be noted that the 1.6/6.1 kb species was challenged by Green *et al* only with the 6.1 kb complementary strand, purified away from any non-specific strands.

A separate issue in nucleic acid biochemistry has been the examination of polymeric species which interact with nucleic acids during strand hybridization processes. Polyethers such as poly(ethylene glycol) have found use in a variety of specific biological or biochemical processes such as viral particle isolation (K. R. Yamamoto *et al., Virology* vol. 40, pp. 734—744 (1970)), nucleic acid purification (B. Alberts in *Methods in Enzymology*, S. P. Colowick and N. O. Kaplans, eds. (Academic Press, N.Y.), vol. 12, pp. 566—581 (1967)), protein purification (K. G. Ingram, *Arch. Biochem. Biophys.* Vol. 194, pp. 59—68 (1977)), mammalian cell fusion (G. Galfre et al., *Nature* vol. 266, pp. 550—552 (1977)) and enhancement of specific enzymatic activity (B. H. Pfeiffer and S. B. Zimmerman, *Nucleic Acids Research* vol. 11, pp. 7853—7871 (1983)). M. Renz and C. Kurz, *Nucleic Acids Research*, vol. 12, 3435—44 (1984) (which may not constitute prior art with respect to the present invention) disclose the use of polyethylene glycol as a volume excluding agent to enhance the rate of hybridization in an immobilized-sample type of DNA probe assay (particularly of the type referred to as a Southern blot). See especially page 3441 of this reference. In this respect other volume exclusion polymers of various types have been shown to enhance the rate of hybridization of matched single strands. See Wetmur et al., *Bipolymers*, vol. 10, p. 601 (1971) (dextran sulfate), Wahl et al., Proc. Nat. Acad. Sci., vol. 76, p. 3683 (dextran sulfate). In general, polymers useful in this regard are non-ionic or anionic water-soluble polymers (including polysaccharides) which do not react with DNA. See S. B. Zimmerman et al, *Proc Nat. Acad. Sci.*, vol. 80, pp. 5852—56 (1983); B. H. Pfeiffer et al, *cited above; Water-soluble Synthetic Polymers* (P. Molyneux, ed.; CRC Press, Inc., Cleveland, OH—two volumes, 1983).

Brief description of the invention

The present invention is based upon the rapid displacement of a labelled polynucleotide from a probe polynucleotide by the target nucleotide sequence of a sample, enabling direct or indirect measurement of label found in or on the displaced labelled polynucleotide (or in some cases the labelled polynucleotide not displaced). This label serves as a reliable and quantitative measurement functionally related to the presence and concentration of target nucleotide sequence in a sample. Accordingly, the present invention includes a method for determining the presence of a predetermined target nucleotide sequence (either DNA or RNA) in the nucleic acid of a biological sample which comprises the steps:

(a) providing a reagent complex of (i) a probe polynucleotide which is capable of base pair binding via hydrogen bonds of purine/pyrimidine base pairs to the target nucleotide sequence, and (ii) a labelled polynucleotide which is bound by base pair binding via hydrogen bonds of purine/pyrimidine base pairs to the probe polynucleotide in a region of the probe polynucleotide at least partially coextensive with the region in which the probe polynucleotide is capable of binding to the target nucleotide sequence;

(b) contacting the reagent complex with a sample under conditions in which the target nucleotide sequence, if present, binds to the probe polynucleotide and displaces labelled polynucleotide from the reagent complex; and

(c) determining the presence (which includes of course determining the amount) either of labelled polynucleotide displaced from the reagent complex or of the labelled polynucleotide remaining in the reagent complex.

The present invention also includes a diagnostic reagent for determining the presence of a target nucleotide sequence in the nucleic acid of a biological sample comprising the reagent complex of:

(i) a probe polynucleotide which is capable of binding via hydrogen bonds of purine/pyrimidine base pairs to the target nucleotide sequence, and

(ii) a labelled polynucleotide which is bound via hydrogen bonds of purine/pyrimidine base pairs to the probe polynucleotide in a region of the probe polynucleotide at least partially coextensive with the region in which the probe polynucleotide is capable of binding to the target nucleotide sequence;

the base pairing between the target nucleotide sequence and the probe polynucleotide being of sufficient cumulative binding strength (as defined below) for the target nucleotide sequence, if present in a sample with which the reagent is contacted, to be able to displace labelled polynucleotide from the reagent complex.

The present invention further includes such a method or a diagnostic kit containing such a reagent

**0 167 238**

complex wherein a volume excluding inert polymer which is non-ionic or anionic (but which is preferably, but not necessarily a polyether) is present in such amounts sufficient to increase the rate of appearance of displaced labelled polynucleotide.

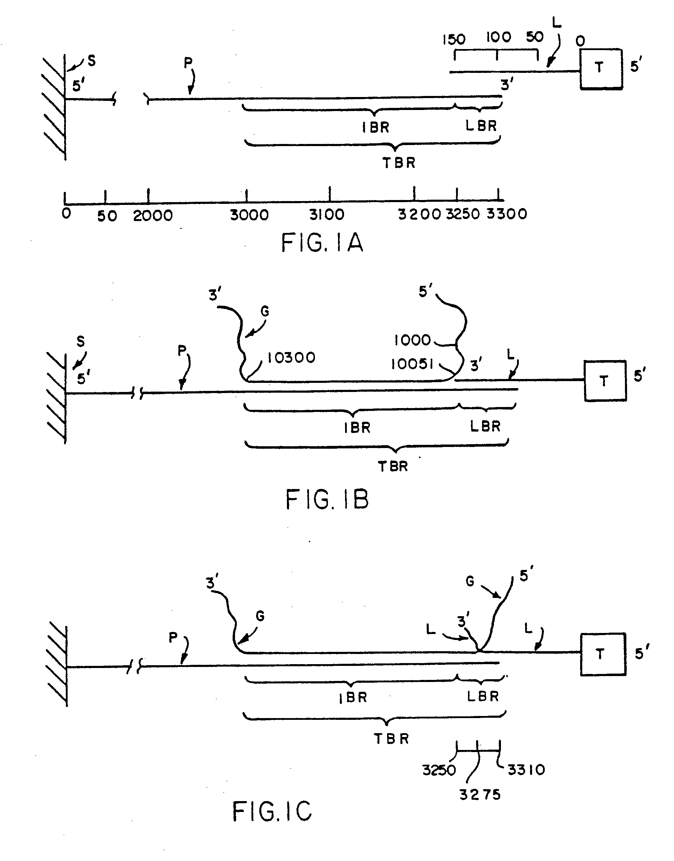

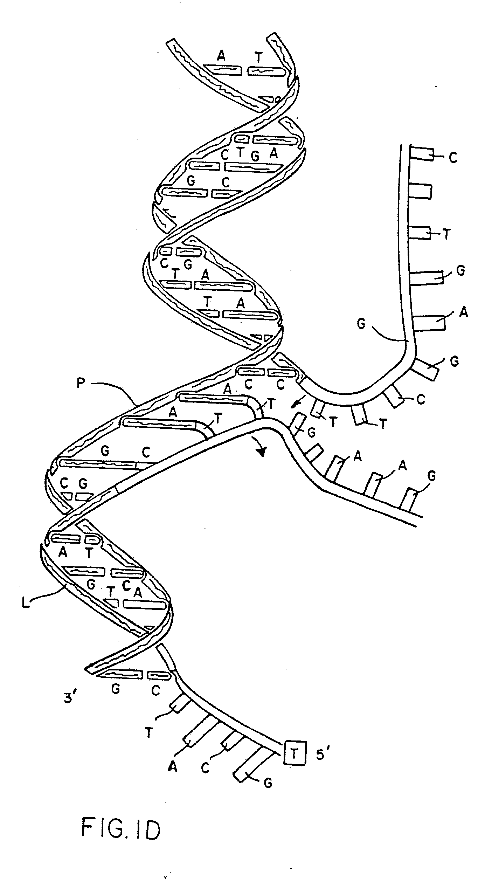

This effect, increasing the rate of appearance of specifically displaced polynucleotide, is sometimes referred to in the description that follows as an increasing rate of displacement. In such event, that nomenclature should not be read as implying that the actual displacement step is expedited on a microscopic or molecular level. In fact, Applicants currently believe that the initial nucleation stage in hybridization of competitor strand to the probe polynucleotide (see Figures 1A and 1B) is the rate-limiting step which is enhanced by volume excluding polymer. If that is true, then no enhancement of the displacement step (cf. Figures 1C, 1D and 1E) is required on a microscopic or molecular level to achieve an increase in the overall rate (compare Figure 1A to Figure 1E).

Brief description of the figures

Figure 1A is a schematic view of one embodiment of the reagent complex of the present reagent useful in the present process;

Figure 1B is a view similar to Figure 1A in which the reagent complex is hybridized with a target nucleotide sequence G of sample nucleic acid;

Figure 1C is a view similar to Figure 1B in which the sample nucleic acid has begun to displace labelled polynucleotide from the reagent complex;

Figure 1D is an enlarged view similar to Figure 1C, in which the helical structure of double-stranded portions is schematically shown;

Figure 1E is a view similar to Figure 1C in which the labelled polynucleotide has been fully displaced from the reagent complex;

Figure 1F is a view similar to Figure 1B of a second embodiment of the present invention in which the immobilized polynucleotide has sequences complementary to and base paired with the sample nucleic acid on both sides of the region where the labelled polynucleotide is bound;

Figure 1G is a view similar to Figure 1A of a reagent complex according to a modification of the first embodiment;

Figure 2 is a view similar to Figure 1A of a reagent complex according to a third embodiment of the present invention, with a sample polynucleotide having the target nucleotide sequence shown prior to hybridization to the reagent complex;

Figure 3A is a view similar to Figure 1A of a reagent complex according to a fourth embodiment of the present invention;

Figure 3B is a view similar to Figure 1C of the fourth embodiment of Figure 3A.

Figure 3C is a view similar to Figure 1E of the fourth embodiment of Figure 3A.

Figure 3D is a view similar to Figure 3A of a modified form of the reagent complex of the fourth embodiment;

Figure 4 is a view similar to Figure 1A of a reagent complex according to a fifth embodiment of the present invention;

Figure 5 is a view similar to Figure 1A of a reagent complex according to a sixth embodiment of the present invention; and

Figure 6 is a view similar to Figure 1A of a reagent complex according to a seventh embodiment of the present invention.

Detailed description of the invention

In this application the following terms are used based on their generally accepted meanings in the field of molecular biology:

Polynucleotide or Polynucleotide Strand refers to a linear polymeric structure of pentose sugars (generally ribose or deoxyribose) linked to each other by 3',5'-phosphodiester linkages, and linked by carbon-nitrogen bonds at the 1-carbon of the sugar to pendant purine or pyrimidine bases such as, but not limited to, uracil (linked naturally to ribose only as rU), thymine (linked naturally to deoxyribose only as dT), cytosine (dC or rC), adenine (dA or rA) and guanine (dG or rG). Polynucleotides thus include strands of deoxyribonucleic acid (DNA) and strands of ribonucleic acid (RNA).

The ends of such Polynucleotide Strands are referred to as the Five Prime (5') end, where the 5-carbon of the pentose is not linked to another pentose (but may bear hydroxyl, monophosphate or other natural or synthetic moieties), and the Three Prime (3') end, where the 3-carbon of the pentose is not linked to another pentose (but may similarly bear hydroxyl, monophosphate or other natural or synthetic moieties).

Complementary Base Pairing or Purine/Pyrimidine Base Pairing refers to the hydrogen bonding between opposite bases pendant on two antiparallel Polynucleotide Strands, which is most energetically favorable for natural DNA when dG is opposite dC and dA is opposite dT. Bases other than the five naturally-prevalent ones also have preferential pairing: for example, 5-methyl-cytosine binds preferentially to guanine. For illustrative purposes, this pairing is shown in many of the Figures by parallel straight lines with complementary strands directed in antiparallel directions (in the 5' to 3' sense). It should be appreciated, however, that the actual geometry of double-stranded segments will normally be helical (the well-known double helix) of various pitches, as schematically illustrated in Figure 1D.

5

Hybridization is used herein to refer to admixing two Polynucleotides under conditions conducive to the formation of double-stranded structures, with Complementary Base Pairing causing such double stranded structures to form where complementary sequences or nearly complementary sequences are present.

The basic components of the method of the invention are a probe polynucleotide (sometimes called herein the probe), a labelled polynucleotide (sometimes called herein tagged polynucleotide or release tag), and the biological sample containing nucleic acid, a portion of which is sometimes called herein the target polynucleotide or target nucleotide sequence. A sample may or may not contain a target nucleotide sequence. In some cases a volume excluding polymer such as a polyether compound is also provided. In some cases a support is also provided, either to which the reagent complex is immobilized via the probe (such that the probe is sometimes called an immobilized probe or immobilized probe polynucleotide), or in other cases as a part of the separation step that may follow displacement as a part of the determination or detecting step. In practicing the process, additional reagents or equipment are frequently required for readout; the term readout refers to the direct or indirect detection of labelled polynucleotide in one or more phases of (usually separated) reaction materials, and especially in a liquid phase by virtue of displacement from the reagent complex and separation of displaced labelled polynucleotide in solution from probe polynucleotides and reagent complexes.

In the practice of the present invention, the probe polynucleotide can be a linear or circular polynucleotide capable of binding specifically through complementary base pairing in at least one region of its purine/pyrimidine base sequence to specific target nucleotide sequences of a sample. This binding may be between DNA and RNA, between DNA and DNA or between RNA and RNA. Accordingly, the probe may either be DNA or RNA. As discussed more fully below, it is generally only a specific region of the probe which binds selectively to the target nucleotide sequence. Other regions of the probe may be of various naturally occurring or synthesized sequences which do not participate in the hybridization reaction with the target nucleotide sequence, but which may play an important role in the present invention, e.g., by serving as a site for attachment to a support or by providing some degree of separation between the support and the region to which the target nucleotide sequence binds, if desired.

Referring to the region of the probe to which the target nucleotide will specifically bind, called herein the target binding region (TBR in the Figures), the binding may be (and preferably is) perfect, in the sense that each nucleotide in the probe sequence finds its correct complementary binding partner (e.g., dA to dT) in the target nucleotide sequence, or may contain some mismatches. At least one portion of the target binding region of the probe is preferably single-stranded in the reagent complex, i.e., it is not complementary to labelled polynucleotide sequences nor self-complementary; this single-stranded region is sometimes called herein the initial binding region (IBR in the Figures) because the target nucleotide sequence can bind to this region of bases without displacing any of the labelled polynucleotide. Such initial binding region of the probe is at least fifteen bases in length, and is preferably at least fifty bases in length. The overall target binding region includes the initial binding region and most or (preferably) all of the labelled polynucleotide binding region (LBR in the Figures and in the discussion below). The length of the overall target binding region is not independently critical, but rather can be considered as a function or sum of the preferred or more preferred lengths of the IBR and LBR portions. Base lengths of the initial binding region of the probe above five hundred are generally not required, but are not significantly disadvantageous in most cases. A suitable lower limit on the length of this region of base pairing for clinical laboratory applications is somewhat dependent upon base sequence of the target binding region of the probe polynucleotide and base composition and other physical factors described below, and especially upon the conditions for intended hybridization, mode of attachment, if any, of the probe to a support, kinetics of hybridization and the readout system employed.

The solid phase to or on which the probe can be immobilized in certain embodiments may be of almost any conventional type, including especially polymeric materials, ceramic materials, walls of a test tube or other container, paper, nitrocellulose or glass. In some forms of the invention, the solid phase consists of natural, modified natural or synthetic particles or beads made of materials such as protein, fixed cells or viruses, or various polymers such as polystyrene, latex or glass.

The means of attachment of the probe to the solid support in certain embodiments of the invention may be simple adsorption, but is preferably some form of specific covalent linkage, ionic bonding, hydrophobic interaction or hydrogen bonding. In the case of covalent linkage, the binding may be direct as by reaction between chemical moieties on the surface of the support (for example, amine or carboxyl moieties) and moieties on the polynucleotide, and especially hydroxyl or phosphate moieties on the end sugar rings of the polynucleotide. Linking agents which are specific to the free secondary hydroxyl normally present at the 3′ end include phosphites, succinic anhydride and phthalamide. Linking agents which are specific to the phosphate normally present on the sugar at the 5′ end (at least for most naturally occurring polynucleotides or products of most cleavage reactions) include carbodiimides such as 1 - ethyl - 3,3 - dimethylaminopropylcarbodiimide, with or without imidazole or 1-methylimidazole. See B. C. F. Chu et al., *Nucleic Acids Research* vol. 11, pp. 6513—29 (1983). Such linkages which are specific to an end or other small portion of the probe, if remote from the target binding region, may permit greater degrees of freedom during the hybridization reaction compared to adsorption or other similar physical or non-specific chemical means of attachment. With such greater degrees of freedom, the minimum length of the target binding region or minimum time for the hybridization to progress to a detectable level may be lowered.

Non-specific covalent linkages include linkages between the substrate and free bases along the chain via moieties such as m-aminobenzyloxy methyl (ABM), m-diazobenzyloxy methyl (DBM) or o-aminophenylthioether (APT). See H. Bunemann et al., *Nucleic Acids Research* vol. 10, pp. 7163—7196 (1982) (two articles). Other exemplary non-specific linking chemistry is described in U.S. Patent 4,286,964 to B. Seed (1981).

In addition to direct covalent linkage, the probe polynucleotide may be indirectly linked in a covalent fashion to the support by a linking or spacer molecule. Examples of indirect covalent linking reagents include spacer reagents which can react by carbodiimide chemistries both with functional groups (for example, esters) on the support and with the phosphate normally present on the 5' terminal sugar of the polynucleotide. Such spacer molecules include the aliphatic diamines used in the above-cited Chu et al. article which, once attached to the terminal phosphate, can then be linked to active groups on the support such as N-hydroxysuccinimide esters. Other spacer molecules include hydroxyalkanoic acids. Still other spacer molecules can also contain a functional moiety such as phenyl ketone which will react directly with a support having hydrazine moieties, forming a resultant hydrazone.

The probe further may be noncovalently linked to the support by interaction of some portion of the probe with affinity reagents that are adsorbed or covalently bound to the support. Examples include (1) immobilization on the support of a short single-stranded polynucleotide which can hybridize to some portion of the probe polynucleotide not overlapping with the region in the probe which is capable of binding to the target nucleotide sequence and (2) binding of a chemically modified probe polynucleotide carrying one or more avidin or biotin moieties to a support having biotin or avidin moieties, respectively, adsorbed or covalently bound to the support. The latter method is based on the high affinity ($K_{diss}$ approximately $10^{-15}$ M) of the small molecule biotin for the protein avidin (or streptavidin).

While the present invention is not limited with regard to the spacings between the point or points of attachment of the probe to a support and the region of the probe polynucleotide which binds specifically to the target nucleotide sequence, it is preferable that this spacing be sufficiently large for the hybridization between target nucleotide sequence and target binding region of the probe polynucleotide to occur such that the target binding region of the probe has sufficient and preferably maximal obtainable freedom of movement during hybridization.

In other embodiments of the invention, the probe polynucleotide is not immobilized on a support, but rather the entire reagent complex is in solution as the reagent is mixed with a biological sample such that hybridization will occur, if at all, in solution. In some of those solution hybridization embodiments, the probe does contain a substituent (such as an affinity reagent, e.g., biotin, or a chemical moiety, e.g., a hapten such as nitrophenol) so as to be immobilizable or separable if desired after hybridization, e.g., by passing the reaction mixture through a porous bed or filter that has streptavidin bound to the support matrix. Such immobilization will cause only displaced labelled polynucleotides to remain in the liquid phase, for subsequent determination. Still other forms of the invention involving solution hybridization include alternative methods of separations such as size exclusion chromatography (see Example 2 below), electrophoresis (see, e.g., Examples 1 below), or other physical separation techniques. Additional forms of the invention, described more fully below in connection with read-out, involve determination of displaced labelled polynucleotide without any separation from complex. Of course many of such determinations without separation apply equally to processes wherein the complex includes an immobilized probe polynucleotide.

Such probe polynucleotides can be manufactured reproducibly in a variety of ways, e.g., cloned in or as a part of a suitable plasmid or viral vector isolated from a bacterium or other suitable microorganism, as a part of the genetic material of a microbe or obtained from any other pertinent biological source. Generally, only a small region of nucleic acid that includes a probe polynucleotide sequence (a portion of which forms the target binding region) will be inserted into any such cloning vectors by recombinant techniques; the remainder, if any, of the cloned insert that is not probe polynucleotide sequence can conveniently be chosen from any polynucleotide sequence heterologous to the target nucleotide sequence. In certain embodiments of this invention, such heterologous sequences can include sequences deliberately selected for specific properties such as the presence of a unique restriction enzyme recognition site. Under some conditions an entire gene or a sequence including an entire gene may be used as an insert, with the vector plus inserted nucleotide sequence either in circular or linear form. In the event that the probe polynucleotide is double-stranded when manufactured, denaturation (either thermally, by adjustment of pH or by disruption of base pairing with other conventional techniques) will normally follow isolation. Cleavage (especially by restriction enzymes or by site-specific chemical cleavage) will normally be used to form double-stranded segments of desired linear form and, if double-stranded circular forms are grown, will precede denaturation. In some cases it may be preferred to purify individual strands from a double-stranded structure either to be used individually as probe polynucleotides or with one as a probe polynucleotide and the other as a precursor for the labelled polynucleotide. This purification can be done by standard techniques such as denaturing gel electrophoresis or affinity chromatography. In some instances, the probe polynucleotide is produced as a single-stranded molecule by replication using

7

single-stranded vectors such as M13 bacteriophage. In some other instances, as described below, the probe polynucleotide and labelled polynucleotide can be manufactured as parts of the same molecule.

The labelled polynucleotide used in the present method and reagent is generally a smaller piece of either DNA or RNA than the probe polynucleotide and has two features of significance: (a) stable but reversible binding to the probe at a specific locus and (b) a label susceptible to detection, especially after displacement. These features are discussed herein separately, followed by consideration of the effect certain types of labelling have on stability and displacement.

The pairing between the labelled polynucleotide and the probe polynucleotide will generally occur over a smaller number of bases than the pairing between the target nucleotide sequence and the probe. In most cases, the bases of the probe polynucleotide to which the labelled polynucleotide specifically binds are a subset of the bases of the probe later binding to the target nucleotide sequence of the sample, and thus represent a portion of what is called above the target binding region of the probe.

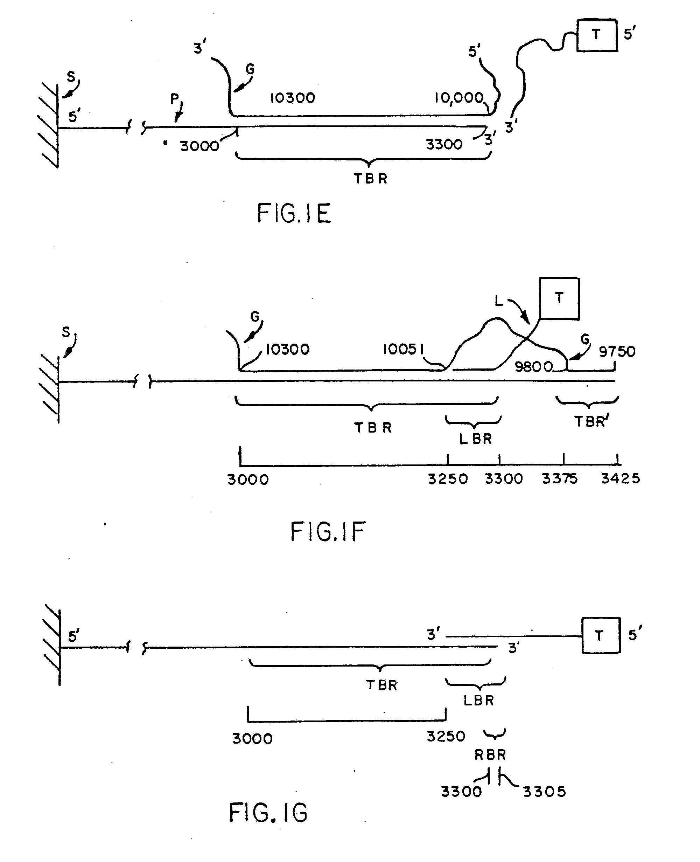

The term labelled polynucleotide binding region (LBR) is used herein to refer to that sequence of bases in the probe to which the labelled polynucleotide is bound in the complex. In preferred embodiments (as illustrated by all Figures) the labelled polynucleotide binding region is totally part of the target binding region (see especially Figure 1A); in other embodiments (see Figure 1G) only a portion (but preferably the major portion) of the labelled polynucleotide binding region is a part of the target binding region. Lengths of labelled polynucleotide binding region outside the target nucleotide binding region of the probe polynucleotide region that are greater than about 15 bases are not preferred because of the potential difficulty of disassociating the labelled polynucleotide from the probe once the only attachment is via base pairing in this region. In some of the preferred embodiments, the region of the probe polynucleotide to which the labelled polynucleotide binds is a subset at or near one end of the larger region of the probe polynucleotide to which the target nucleotide sequence of the sample subsequently binds. In some such embodiments, if the probe is immobilized in the reagent complex, the aforementioned one end of the larger region is at or near an end of a linear probe polynucleotide (as illustrated in Figure 1A and discussed below; note, however, that the other end of the TBR may also be used as an LBR).

The present reference to a labelled polynucleotide and a probe polynucleotide as distinct entities should not be understood, however, to be a requirement that they are linked solely by complementary base-pairing or that there is necessarily only one labelled polynucleotide attached to each probe polynucleotide or that there is only one labelling moiety (tag) per labelled polynucleotide. Other forms of attachment of probe to labelled polynucleotide may also be present, but are generally not preferred. In embodiments without a separation, severing such other attachment of probe to labelled polynucleotide may or may not be required, depending upon whether or not displaced labelled polynucleotides still attached by such other means appear in the detection method as if they were totally displaced. Severing such other attachment before, during or after displacement but, in any event, before the determination step is preferred. Multiple labelled polynucleotides on a single probe strand (as illustrated in Figure 6) is a form of the invention which may have special application when greater numbers of displaced tags per displacement event are desired.

The size of the labelled polynucleotide binding region of the probe is not itself critical to the present invention, because wide variations in this size can be compensated for, e.g., by modifying the temperature of the displacement step (contacting step (b) described above) and by the size of the region of the probe to which the sample binds. A generally preferred size for labelled polynucleotide binding region (and the corresponding base sequence of the labelled polynucleotide) is at least about 15 bases, preferably at least 20 bases, and more preferably at least about 25 bases. A preferred range is about 20 to about 1000 bases (more preferably about 20 to about 500 bases, and most preferably about 25 to about 200 bases). Labelled polynucleotides with unusually short pairing segments (giving regard to factors such as GC base content) may dissociate from the probe in a non-specific manner if the temperature of the displacement step is too high (also giving consideration to factors such as salt concentration which affect melting temperatures). See D. Freifelder et al, *Biopolymers*, vol. 7, pp. 81—93 (1969). There is no essential advantage in unusually long pairing segments (e.g., over 1000 bases). Such long pairing segments are sometimes less preferred because the overall target binding region of the probe could then become longer and require necessarily longer target nucleotide sequences for successful displacement of the labelled polynucleotide. The binding portion of the labelled polynucleotide may, if too long, no longer easily be manufactured by certain techniques presently available or that are best suited for small polynucleotides: e.g., organic chemical synthesis of the entire labelled polynucleotide. Organic chemical synthesis is generally easier at present for labelled polynucleotide binding regions of less than about 100, and especially less than about 60; however, improvements in such synthetic techniques could make longer sequences easy to make as well.

The minimum length of labelled polynucleotide (and also of labelled polynucleotide binding region) is related primarily to reagent complex stability. Factors other than length which affect this stability include GC content (whose effect on melting temperature is well known) and internal base pair mismatches. Melting temperature is a useful way to establish an effective length for sequences having one or more internal base pair mismatches. As an example, a sequence of 30 bases having one internal base pair mismatch (and thus only 29 exact pairs) may perform (at least with regard to reagent complex stability) effectively like a shorter exactly matched sequence; the degree of departure from behavior as a 29 base pair sequence will depend upon the position of any base pair mismatches and the base change that has been made. Effective length can be expected to differ depending upon whether the mismatched pair is purine/purine, purine/pyrimidine or pyrimidine/pyrimidine. Any effective length can be empirically determined, however, by melting temperature experiments in which a series of probe/labelled polynucleotide complexes are subjected to various temperature regimes, as described in articles such as R. L. Ornstein and J. R. Fresco, *Biopolymers*, vol. 22, pp. 1979—2000 (1983). By determining a melting temperature of a complex of probe with a labelled polynucleotide containing base pair mismatches, and comparing that value with melting temperatures of complexes with slightly shorter lengths of pairing (but perfect base pair matching within the paired region) on the same probe polynucleotide, the effective pairing length of the labelled polynucleotide with base pair mismatches can be estimated and applied to the above preferred and more preferred ranges. The above correlations based upon melting points are intended, however, primarily as an easy estimation tool. The actual degree of preference for a given labelled polynucleotide binding region is based both on how well the labelled polynucleotide actually stays in the complex during storage of the reagent or contact with non-homologous nucleic acids under use conditions and on how well the labelled polynucleotide is displaced by nucleic acids having the appropriate target nucleotide sequence. While base pair mismatches are permitted, they are in general not preferred and, when present, generally comprise no more than two fifths and preferably comprise no more than one tenth of the region of pairing (e.g., a preferable maximum is three mismatches and twenty-seven perfect matches in a 30 base pair long region of labelled polynucleotide/probe polynucleotide pairing).

The labelled polynucleotide may contain regions of nucleotides, in addition to the pairing region, which do not specifically bind to the probe polynucleotide. Such regions may serve to link the pairing region to the detectable tag, may themselves be tagged (e.g., by radioactive labelling or by covalent attachment to an indirect marker such as avidin, steptavidin or biotin) or merely be present without any particular function. The labelled polynucleotide may itself be linear or circular and may be (but is preferably not) double stranded in regions other than the pairing region. The labelled polynucleotide is preferably not circular when the probe polynucleotide is circular because of possible topological constraints on hybridization of two circular nucleic acid molecules and possible constraints on hybridization with the target nucleotide sequence.

One or more detectable tags may be generally located (using conventional techniques) at one or more of several points along the labelled polynucleotide (especially if the tag is a radionuclide or biotin or the like), only at one end or only at one specific internal location on the labelled polynucleotide (e.g., at a purine or pyrimidine base not involved in base pairing to the probe polynucleotide). Provided that there is at least one region of the labelled polynucleotide unpaired in the reagent complex, the tag is preferably present or concentrated on or within such unpaired region. Directly detectable tags which may be used include radionuclides (especially phosphorus-32, sulfur-35, carbon-14 or iodine-125 labelled nucleotides), fluorescent compounds (such as fluorescein or rhodamine derivatives attached to the free end of a labelled polynucleotide or to one or more of the unpaired bases of a labelled polynucleotide) or moieties directly detectable by other means (including being cleaved off) such as the moiety nitrophenol detectable colorimetrically or otherwise. Indirectly detectable tags include those modifications that can serve as antigenic determinants, affinity ligands, antigens or antibodies recognizable through immunochemical or other affinity reactions such as described in EPA 63,879, WO 83/02277 and EPA 97,373, referenced above and exemplified by biotinated nucleotides present in or added onto the labelled polynucleotide (such as by the enzyme terminal deoxynucleotidyl transferase which will add multiple nucleotides at the 3' end of the labelled polynucleotide in the absence of a template strand).

Other indirect tags include enzymes attached to a labelled polynucleotide (especially at a free end remote from the region paired to the probe) whose presence can be determined after displacement and separation steps of the embodied method by addition of the substrate for the enzyme and quantification of either the enzymatic substrate or, preferably, the enzymatic reaction product. Similarly, the tag may be an apoenzyme, co-enzyme, enzymatic modifier, enzymatic cofactors or the like, with the other necessary reagents usually added after displacement (and, in certain embodiments, after separation) along with the appropriate enzymatic substrate, after displacement (and, in certain embodiments, separation). Of course, if the enzymatic reaction cannot occur with all but one component present (e.g., the substrate), then these other reagents may be present in solution during the contacting (displacement) step (b) described above.

Multiple detectable tags can be added in manufacturing the labelled polynucleotide such as by using a terminal deoxynucleotidyl transferase enzyme. Multiple labelled polynucleotides, e.g., one containing the enzyme (or apoenzyme) and one containing the coenzyme, can also be used. One form of attachment of an enzyme to the labelled polynucleotide is via affinity reagents, e.g., streptavidin to biotin. Such a binding form can be used in various embodiments, for example wherein the complex is prepared by hybridizing a biotin-labelled polynucleotide to the probe and then binding a streptavidin-enzyme conjugate to the biotin prior to the contacting (displacement) step (b), described above. Furthermore, a moiety interacting with the detectable tag in the complex may be present on the probe.

Most forms of detectable tags, especially if remote from the pairing region of the labelled polynucleotide to the probe, will have little or not effect on the strength of base pairing between the labelled polynucleotide and the probe polynucleotide, as evidenced (for testing purposes) by little or no diminution of the reagent complex melting temperature and, more importantly, by negligible effects on the hybridization reaction between any target nucleotide sequence and the probe polynucleotide. Some forms

9

of labelling, such as covalently bound biotin on nucleotides of the labelled polynucleotide in the base pairing region and such as a large enzyme molecule or fluorescent moiety linked to nucleotides in or near the pairing region, may have an appreciable effect on reagent complex stability. Such effect generally will be to destabilize the labelled polynucleotide/probe polynucleotide binding (and thus lower its melting temperature). That effect may be somewhat beneficial in speeding up displacement, but can cause increases in non-specific dissociation or "fall-off" of the labelled polynucleotide. Such non-specific "fall-off" can usually be reduced, however, by lowering the temperature during the displacement step, increasing the length of the labelled polynucleotide binding region, or other such modification of the physiochemical properties of the system.

Forming a reagent complex between an immobilized probe polynucleotide and a labelled polynucleotide (such complex being provided in the present process and being in the present reagent) may involve attachment of the probe to a support (as described above) either before or after hybridization of the labelled polynucleotide to the probe polynucleotide. Affinity or other chemical reagents may be used for mediating or participating in such attachment. If the immobilization is completed after hybridization of the labelled polynucleotide to the probe polynucleotide, then the linking moiety or a part thereof can already be attached to the probe.

Generally, the formation of such an immobilized complex will be followed by washing off unbound labelled nucleotide, and the conditions of such washing may be designed to also remove labelled polynucleotides that are only slightly bound to the probe (e.g., through less than about fifteen complementary bases elsewhere on the probe, instead of through the larger number of complementary bases at the desired binding site) or are adsorbed to the support. Probe polynucleotides and complexes of probe polynucleotide with labelled polynucleotide that are only marginally attached to the support may also be removed during this washing step. The washing should preferably be under sufficient conditions and for a sufficient time to substantially eliminate the non-specific background signal due to labelled polynucleotides (with or without probe polynucleotide) separating from the support independently of specific displacement during the displacement step of the present method. One can also use a reagent (e.g., protein) to block any non-specific binding sites on the support.

In the manufacture of reagent complexes which are in solution when used, it is frequently also desirable to separate labelled polynucleotides which have not bound to probe polynucleotides from product reagent complexes (in some instances unbound probe polynucleotides may also be removed). Such separation may be by size alone (e.g., by size exclusion chromatography) if, as is frequently the case, the labelled polynucleotide is much smaller than either probe polynucleotides or reagent complexes. Such separation may also be based upon the double-stranded nature of at least one portion of the reagent complex (at the labelled polynucleotide binding region of the probe) where such a double-stranded region is not likely to be present in either the labelled polynucleotide or probe polynucleotide, both of which would be expected to be in single-stranded form except for very small internal binding regions. This property renders reagent complexes separable from unbound labelled polynucleotides by, e.g., affinity chromatography using double-strand specific anti-nucleic acid antibodies or hydroxylapatite chromatography.

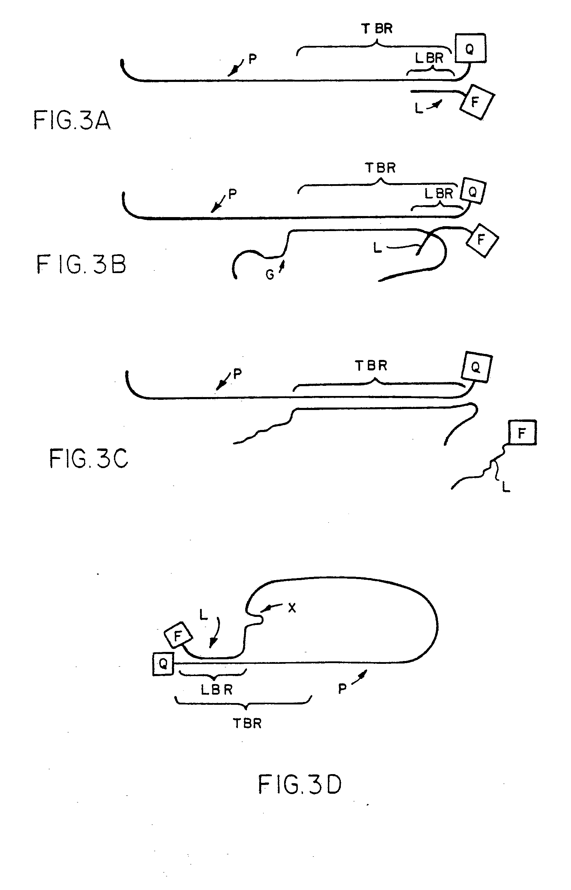

In some instances, the labelled polynucleotide and probe polynucleotide can be part of the same polynucleotide chain. For example, a linear single-stranded DNA molecule can be constructed from an M13 bacteriophage which contains a cloned DNA insert with inverted repeats. These inverted repeats, which are capable of forming a double-stranded region due to their complementarity, would include the labelled polynucleotide and the labelled polynucleotide binding region. Sequences located between or adjacent to these repeats would include the initial binding region, located adjacent to the labelled polynucleotide binding region. Unique restriction enzyme cleavage sites, e.g., the M13mp7 polylinkers or modifications thereof, located outside of the inverted repeats and the initial binding region, could be cleaved to release the cloned insert from the single-stranded M13 vector backbone (c.f. G. A. Ricca, J. M. Taylor & J. E. Kalinyak, *Proc. Nat. Acad. Sci.* U.S.A., vol. 79, pp. 724—728 (1982)). An additional small inverted repeat sequence, containing a restriction enzyme cleavage site (e.g., the M13mp7 polylinker) could be placed between the labelled polynucleotide repeats. Cleavage at such a site (e.g., X of Figure 3D) would leave only complementary purine/pyrimidine base pairing to hold the labelled polynucleotide attached to the now distinct probe polynucleotide and would provide free ends on the probe polynucleotide through which attachment to a solid support could be mediated if so desired. Any tag or tags may be added to the labelled polynucleotide at this point if not already present.

If the volume-excluding polymer used in some forms of the present invention is a polyether, it may be a simple poly(alkylene oxide) such as those of the formula $H(O—R)_n—H$ where R is alkylene of 1—6 carbons and n is an integer (on a weight average basis) of 15—1000. R can be methylene, 1,1-ethylene, 1,2-ethylene, 1,2-propylene 1,4-butylene, 1,2-butylene, 1,2-hexene (2-butylethene) or similar divalent saturated hydrocarbon moieties, and can be mixed within a single molecule. Preferably R is 1,2-ethylene or 1,2-propylene as in poly(ethylene glycol) (PEG, also called poly(ethylene oxide) or PEO) or as in poly(propylene glycol) (PPG, also called poly(propylene oxide) or PPO). The integer n in such instances represents the weight average molecular weight divided by the monomeric unit molecular weight; such monomer unit molecular weight for PEG is 44 and for PPG is 56. For PEG, weight average molecular weights are preferred empirically to be at least about 1500, preferably at least about 2500 and more preferably between about 3000 and about 20,000. Accordingly, n (on a weight average basis) is preferably at least 36, more preferably at least 60 and most preferably between about 70 and about 690. A similar set of preferred ranges for n in PPG is expected.

Also included as suitable polyether polymeric agents in the present invention are condensation products of ethylene oxide, propylene oxide or other alkylene oxides on various moieties such as diols, triols, sugars or acids. Such materials are well known in the art of non-ionic detergents and may be effective in the present invention provided that the molecular weight (and water solubility) is sufficient to enhance displacement without unduly enhancing non-specific dissociation of labelled polynucleotide from the probe polynucleotide.

In certain broader forms of the invention, however, other chemically inert, water-soluble nonionic or anionic polymers may be used as the volume excluding polymer in place of or in conjunction with the above polyethers. Examples include polyacrylates, poly(vinyl acrylates), derivatized and underivatized polysaccharides, polyphosphoric acid, and various copolymers containing pendant carboxyl, sulfonate, sulfate, phosphate, or other anionic moieties. A material shown to have limited beneficial effects is Ficoll, MW 400,000 (a cross-linked reaction product of sucrose with epichlorohydrin having free hydroxyls; Ficoll is Pharmacia's trademark). A class of suitable non-ionic volume excluding polymers is thus the reaction products of a sugar with epichlorohydrin.

Certain volume excluding polymers are preferably used in the total method of the invention, involving both the contacting (displacement) step and the determining (detecting) step only in forms in which their beneficial effects upon the displacement reaction are not outweighed by deleterious effects upon the determination step or a portion of the determination step. For example, in those embodiments wherein the determination step includes a separation (e.g., gel chromatography, size exclusion chromatography, affinity chromatography or electrophoresis), the presence of certain volume excluding polymers may have deleterious effects. Dextran sulfate, while believed to be useful in promoting displacement, was observed in several experiments to have deleterious effects upon electrophoresis (under the particular conditions tested) and in more limited experiments to have deleterious effects upon size exclusion chromatography (under the particular conditions tested). It is believed, however, that one can find, through routine experimentation, suitable polymers for any particular embodiment of the present method and can find, through routine experimentation, suitable detecting or determining conditions for using any polymer which has the effect of promoting the hybridization and displacement process.

The actual contacting or displacement step with sample material (potentially containing nucleic acids that may include the target nucleotide sequence) will normally be under conditions of temperature, ionic strength, polyether polymer (or similar volume-excluding polymer) concentration and time less stringent (and thus less conducive to non-specific uncoupling of the labelled polynucleotide) than the above washing step. A desirable temperature range during the contacting step is from about 15°C to about 90°C, depending upon the solution ionic strength and other additives affecting melting temperature; the most efficient temperature will be one at which a maximum or near maximum rate of hybridization of sample nucleic acids to probe occurs. In certain cases, however, a more convenient (generally lower) temperature such as from near ambient temperature (15—25°C) up to physiological temperatures (37—40°C) may be used. As described in a number of literature references (e.g., J. G. Wetmur and N. Davidson, *J. Mol. Biol.,* vol. 31, pp. 349—370 (1968) and C. Minson and G. Darby, *New Developments in Practical Virology,* vol. 1, pp. 185—229 (Alan Liss, Inc., N.Y., 1982)), hybridization rate is also a function of pH and sample nucleotide concentration. Also, in addition to the application of a polyether polymer in the preferred assay methods described herein, other water soluble volume excluding polymers such as dextran sulfate can be used in conjunction with the polyether polymer. Enzymes and other proteins which affect the displacement process (such as the *E. coli* ATP-dependent rec A protein) may further be present, however, based upon their beneficial effects described in EP—A—164876.

Proportions, amounts and concentrations of reagent complex and, if used, polyether polymer are not independently critical, but it is generally desired that the total hybridization mixture of sample and reagent complex be as concentrated as feasible. In most instances, probe polynucleotides bearing binding sites for the target nucleotide sequence will be expected to be present in ten-fold or more molar excess (possibly hundred-fold or more molar excess) of any anticipated level of target nucleotide sequence in the sample. The sample itself may include nucleic acids which preferably should be completely or partly in solution (separated from membranes and the like) and in single-stranded form for the hybridization step of the assay. The presence of the unlabelled complement of the target nucleotide sequence (by virtue of denaturation of double-stranded sample DNA) could represent an interference. This interference is likely to be minor in at least the preferred forms of the invention due to the usual molar excess of reagent over sample strands having the target nucleotide sequence or its complement; in hybridizations involving immobilization of the probe selectively (before or after displacement), displaced labelled polynucleotide will be and will remain in the solution phase and be subsequently determined, whether or not such displaced labelled polynucleotide has rehybridized with complementary segments of the sample nucleic acid. In many solution hybridizations this interference may also be minimized by kinetic effects.

In general, the optimal proportions of polyether polymer, if used, can be expressed as a weight proportion (weight/vol or weight %) of the total aqueous phase during the displacement reaction. Such proportion will generally be from 2% up to the solubility limits of the polyether polymer and will preferably

# 0 167 238

be between about 5% and about 20% (weight %). For particular reagent complex and displacement conditions, optimal proportions and appropriate polymer molecular weight can be further determined by routine experimentation as illustrated in Examples 13 and 14.

The basis for the volume exclusion phenomenon, incompatibility of two water soluble polymers (in the present case being the nucleic acid and the volume excluding non-ionic or anionic polymer), is summarized in P. Molyneux, *Water-Soluble Synthetic Polymers: Properties and Behavior*, vol. 2, pp. 167—170 (1984) (see also references cited therein).

In general, displacement reactions using volume excluding polymers should require no more than three hours, generally under one hour and desirably less than thirty minutes to occur to a sufficient extent for detection. Conditions often can be adjusted to achieve substantial completion of strand displacement reactions within these times. Longer incubation times are not necessarily disadvantageous, however. It is believed that the rate-limiting step is a sample or target nucleotide sequence finding a complementary sequence of the probe polynucleotide (cf. C. Green and C. Tibbetts, *Nucleic Acids Research*, vol. 9, pp. 1905—18 (1981)); once target/probe hybridization begins to occur, displacement of labelled polynucleotide is expected to occur from each individual reagent complex in under one minute and frequently less than one second.

In some of the forms of the invention in which the complex is initially on a solid support, the liquid phase containing displaced labelled polynucleotide may be separated therefrom as a part of the determination step. If the complex is in solution, some forms of the invention involve treatments after the contacting (displacement) step to fix reagent complexes still present (and, unavoidably, in some cases other forms of the probe including those hybridized to a target nucleotide sequence) to a solid support, followed by a similar solid/liquid separation. Such separation of the solid phase containing bound complex from liquid phase containing displaced labelled polynucleotide may be by physical means such as chromatography, filtration, centrifugation, magnetic attraction or decantation. The solid phase may include magnetic or other separable particles that are attracted or otherwise physically removable from the liquid phase. Furthermore, complete separation of the two phases is not required; an aliquot of the liquid phase following partial separation may be removed for label determination, leaving the solid phase admixed with the remainder of the liquid phase. If such a separation occurs or is used, determination (detecting) of the presence and frequently the quantity of labelled polynucleotide present may be conducted upon either phase, but is preferably conducted upon the liquid phase. Determination of the label in the liquid phase as a measure of the presence and quantity of displaced labelled polynucleotide typically has the advantage of generally lower background signals. Any background signal will be largely that caused by non-specific fall-off from a solid support as described above, by non-specific dissociation of labelled polynucleotide strands from reagent complexes, or by imperfect preparation of reagents. Additionally, the absence of target nucleotide sequence in the sample results in the proportional absence of signal above background levels from labelled polynucleotide in the liquid phase. By contrast, if detection is of the label associated with the solid phase, a negative result is indicated by unreduced levels of labelled polynucleotide. Especially when a high molar excess of reagent complex over sample nucleotide sequences over anticipated target nucleotide sequences is used, measurement of the label remaining on the solid support results in substantially reduced sensitivity.

In some embodiments of the invention, no post-displacement separation occurs, and determination can still be made of displaced labelled polynucleotides. Some such determination (detecting) steps involve a change in the signal detectable from a tag by virtue of displacement, including those signals affected by proximity of the tag to a signal-affecting moiety elsewhere on the labelled polynucleotide or on the probe polynucleotide (see description of Figure 3A—3D, below).

In other forms, especially with immobilized probes, the interaction can be between two types of tag moieties. One type of detectable tag can be on labelled polynucleotides hybridized to immobilized probe polynucleotides at one location on a solid support. A second type of tag may be on labelled polynucleotides which are hybridized to immobilized probe polynucleotides at a remote location on the same solid support or on a separate solid support in close physical proximity. The second type of tag may also be otherwise directly attached to some remote location of the probe or of the same labelled polynucleotide or of the solid support. In all such cases involving two types of tags, the two different tags can interact only if at least one labelled polynucleotide (tag) is displaced. Such interaction is especially applicable to apoenzyme with coenzyme: e.g., apoglucose oxidase with flaving adenine dinucleotide (FAD).

The process and reagent of the present invention can be used for the detection and determination of a variety of target nucleotide sequences in a variety of concentrations. In particular, microorganisms including infectious agents whose nucleic acid (genomic or otherwise) can be targeted include pathogenic viruses, bacteria and fungi; e.g., cytomegalovirus or *Neisseria gonorrhea*. Exemplary genetic disorders or conditions which can be targeted include $\beta$-thalassemias, $\alpha_1$-thalassemias, cri du chat syndrome and some retinoblastomas. The present process and reagents are applicable to detecting genetic disorders or variations primarily when a multi-base nucleotide deletion, insertion, substitution or transposition is involved in distinguishing the target nucleotide sequence from the closest sequence present in samples intended to be read as negative for the target sequence. To the extent that the present invention is applicable to genetic disorders due to single base mutations, if at all, the complement of the substituted base or other point of mutation is desirably part of the target binding region of the probe polynucleotide,

12

with the location of that base within the region likely to affect the selectivity of the method. Changes or differences in the expression, activation or rearrangement of structural genes, regulatory genes or oncogenes can be detected by the present method. For monitoring gene expression, mRNA would be targeted. Other perturbations in the expression of structural genes can be similarly detected. The present process can also be applied to histocompatibility typing for tissue transplantation, determination of antibody resistance genes in microorganisms, and to the screening of food, medicinal and water samples for specific infectious agents or other microorganisms.

Selecting a target sequence for a particular test involves determining a sequence which is unique or relatively unique to the target organism or condition. Such target sequence would be used to develop the target binding region (which is complementary thereto) and then a labelled polynucleotide of appropriate length would be developed to bind to a part of the target binding region. In some instances multiple reagent complexes targeting different parts of the nucleic acid of the same organism or condition may be used in order to impart improved specificity when any particular target sequence is only relatively unique.

One embodiment of the present invention is illustrated, for purposes of understanding, by reference to attached Figures 1A—1E, in which is shown an immobilized probe polynucleotide P, 3300 nucleotides in length. Numbering from the 5' end, the region from nucleotide 3000 to nucleotide 3300 represents the target nucleotide binding region (TBR). For simplicity, it is assumed that the first nucleotide of the probe is directly attached to a support S. As illustrated in Figure 1A, the labelled polynucleotide L consists of 150 bases to which a tag T is attached at the 5' end. Of these 150 bases, 51 (from base number 100 to base number 150) bind specifically to the labelled polynucleotide binding region (LBR), bases 3250 to 3300, of the probe. Accordingly, when the immobilized probe consisting of probe polynucleotide P attached to support S is incubated with a solution of labelled polynucleotide L under normal hybridization conditions, a complex will be formed as shown in Figure 1A with labelled polynucleotide L attached at the end of the probe polynucleotide P via base pairing between bases 3250—3300 of the probe P and bases 100—150 of the labelled polynucleotide L.

In use, the illustrative complex is contacted with a biological sample under hybridization conditions where the sample contains a nucleotide sequence with bases 10000 to 10300 of this sequence which corresponds to and binds selectively to the target binding region TBR of the probe. Under proper hybridization conditions, the sample polynucleotide G would first bind (form base pairs), as illustrated in Fig. 1B, from base 10051 to base 10300 of G and base 3000 to base 3249 (the initial binding region or IBR) of probe P. The IBR in the reagent complex shown in Fig. 1A is entirely single stranded. It should be appreciated that thermodynamic considerations favor the formation of double-stranded moieties such as shown in Fig. 1B over single-stranded moieties. It is believed, however, that this initial hybridization is the rate-limiting step and that the present use of volume excluding polymers facilitates or speeds up this step under many conditions. Thereafter, an equilibrium is created at bases 3250 to 3300 (LBR) of the probe polynucleotide P between binding to the labelled polynucleotide L and binding to bases 10000 to 10050 of the sample polynucleotide G. Under these conditions, a rapid zippering and unzippering will occur, as represented by the differences between Figure 1B on the one hand and Figures 1C on the other hand, along with random migration of the branch point along the probe polynucleotide. In such event, when additional bases of the sample polynucleotide G bind to the probe polynucleotide beyond probe base 3249, corresponding bases of the labelled polynucleotide L are displaced, creating a free end. Under the conditions described herein, this zippering and unzippering can occur in either direction; but the favored event will be eventual shifting of the point of attachment to the right in Figure 1C. Figure 1D shows the stage of near total labelled polynucleotide strand displacement in somewhat enlarged and more graphic form from base number 3275 on the probe P toward base number 3300, at which point the labelled polynucleotide L is totally displaced from the probe P (cf. Figure 1E). It should be appreciated that, merely upon random zippering and unzippering in both directions, removal of the labelled polynucleotide L will be favored in that Figure 1B represents the furthest to the left that the labelled polynucleotide L can displace the target nucleotide sequence of sample polynucleotide G. As long as some substantial region of the initial binding region IBR exists in which a sequence of nuelcotides of the probe polynucleotide P binds selectively to the target nucleotide sequence, but not to the labelled polynucleotide, displacement of the sample polynucleotide G completely from the probe will be a rare and reversible event.

An important parameter in optimizing the present method and reagent is the length and nature of the base pairing between the labelled polynucleotide and the probe polynucleotide. As compared to the 51 bases of exact pairing illustrated in Figure 1A, modification may be made either by shortening or increasing this length, by introducing mismatches or loops in either strand or by selecting between DNA and RNA for either or both strands so as to effect the rate of spontaneous (and presumably also specific) dissociation. In this regard, certain differences can be achieved when the target nucleotide sequence is RNA and the labelled polynucleotide is DNA, whether the probe polynucleotide P is either DNA or RNA. This is because, in general, RNA-RNA binding is strongest per base pair, DNA-RNA base pairing is of intermediate strength and DNA-DNA base pairing is of the least strength per base pair. If, however, the target binding region (TBR) is larger than the labelled polynucleotide binding region (LBR), then the labelled polynucleotide can be RNA and still be displaced by a DNA target nucleotide sequence. Targeting RNA may be required if, for example, the target microorganism is an RNA virus or the target condition is one of altered gene expression.

One means of reducing the binding strength in the region in which the labelled polynucleotide is bound to the probe polynucleotide is the introduction of individual base mismatches into the labelled polynucleotide. Assuming that the probe nucleotide sequence will be chosen to pair exactly or nearly exactly to the target nucleotide sequence, such mismatches can be considered as mutations or individual base substitutions in the labelled polynucleotide compared to a similar polynucleotide segment of the target nucleotide sequence which has exact (or nearly exact) binding. Such mutation or substitution may include the substitution of a natural or chemically modified nucleotide for a given natural nucleotide such as the following: G for A (to produce the opposite pair dG-rU, dG-dT, rG-dT or rG-rU), A for G, 5-methylcytosine for C, 5-hydroxymethylcytosine for C, gentibiosyl - 5 - hydroxymethylcytosine for C, or 5-bromouracil for A. In many preferred embodiments, such mutations involve the substitution of one naturally occurring nucleotide for another nucleotide. In certain such embodiments, the substitution involves the substitution of a pyrimidine for a purine, leading to a mismatched pair that has become a pyrimidine-pyrimidine pair. Because pyrimidines occupy less space than purines, such individual pyrimidine-pyrimidine mismatches can have a minimal effect upon adjacent nucleotide pairings. Purine-pyrimidine mispairings (for example, A being positioned opposite to C, or T or U being positioned opposite to G) are somewhat more space filling, but are still generally less space filling than the positioning of two purines (A-A, A-G or G-G) apposite each other. Counteracting the space-filling effect, however, is a stacking energy effect which runs in the opposite direction: generally, purine/purine base pairs display a lower free energy of stacking, purine/pyrimidine base pairs somewhat more and pyrimidine/pyrimidine base pairs somewhat more still. See R. L. Ornstein & J. R. Fresco, *Biopolymers*, Vol. 22, pp. 1979—2016 (1983) (two articles). The net effect, in terms of effect on melting temperature of the reagent complex, in terms of effect on stability of the reagent complex and in terms of effect on the displacement kinetics, will primarily represent a combination of these two counteracting effects, such that experimentation may be required to determine which, if any, mismatches may be preferably incorporated into the labelled polynucleotide for a particular probe after the position of the target nucleotide region and labelled polynucleotide region are fixed.

In addition to point substitutions, mismatches can be created either by inserting a short sequence in the labelled polynucleotide L which does not correspond to the probe nucleotide sequence (e.g., inserting a series of A's between bases 125 and 126 of labelled polynucleotide L in the example illustrated in Figure 1A) or by deleting a portion (such as by deleting bases 120—130 of labelled polynucleotide L in Figure 1A). Insertions in the labelled polynucleotide will generally form a single-stranded loop or cruciform structure of the labelled polynucleotide; deletions therein will generally form a single-stranded loop or cruciform structure of the probe polynucleotide.

Such substitutions, deletions or insertions will have the effect of destabilizing the binding between the labelled polynucleotide L and the probe polynucleotide P such that the displacement of the labelled polynucleotide may be increasingly favored. It is important, however, to avoid to the extent possible nonspecific displacement of the labelled polynucleotide L from the probe polynucleotide P in the absence of the target nucleotide sequence. The minimum length of binding between the labelled polynucleotide L and the probe polynucleotide P which is sufficient to minimize such dissociation or falling off will be dependent on a number of factors including, especially, the conditions such as pH and temperature of hybridization, the mode of attachment of the probe polynucleotide to the support (e.g., end attachment versus nonspecific adsorption), the extent of destabilizing substitutions (or deletions or additions), the duplex base sequence at the region of hydrogen bonding and whether the binding is between RNA and RNA, DNA and RNA or DNA and DNA. The optimal conditions in a particular instance can be determined empirically with routine experimentation based upon the general teachings of the present disclosure. Such experimentation may involve melting temperature experiments with samples lacking the target nucleotide sequence for purposes of estimating stabilities, and then displacement experiments for final optimization.

In considering the geometric relationship between the region where the labelled polynucleotide binds to the probe polynucleotide (LBR) and the region where the target nucleotide sequence binds to the probe polynucleotide (TBR) a configuration such as that illustrated in Figures 1A—1B may be used, with the labelled polynucleotide binding region being a subset of and at the end of the target binding region distal to the solid support. A common end of the TBR and the LBR (probe nucleotide 3300 in Figure 1A) may also be (or be near to) one end of the probe polynucleotide. There are, however, no great disadvantages in having the probe polynucleotide extend beyond this point in a sequence of a nonspecific nature. There may be situations in which it is desirable to have a probe polynucleotide continue beyond this pairing region and extend to a point of attachment of a tag (different from the tag T on labelled polynucleotide L) which is to be released subsequently by other techniques. Furthermore, the two tags (one on the labelled polynucleotide, the other in the probe polynucleotide) may interact, with the interaction being detected as a part of the read-out. It may be preferred in certain embodiments of the invention that the labelled binding region be near or at the end of the target binding region nearest the support.

Situations which are included in the present invention, but which may be less preferred include extension of probe polynucleotide P beyond the LBR (that is, for example, for the 125 bases beyond base 3300 in Figure 1F) with a region (such as bases 3375—3425) which binds selectively to a portion of the sample nucleotide (and especially a region such as bases 9750—9800 of the sample polynucleotide G). In such an event, the sample polynucleotide G could bind both at bases 3000—3249 of the probe polynucleotide P and at bases 3450—3500 of the probe polynucleotide P and overlay the labelled polynucleotide L. Such a structure, represented generally as a "triple-stranded" region or D loop (see C. Green and C. Tibbetts, *Nucleic Acids Research*, vol. 9, no. 8, pp. 1905—18 (1981), especially page 1912), might not cause displacement of the labelled polynucleotide L in certain topologies and thus would represent a potential specific binding event without a signal generation. While displacement at bases 3250—3300 of the probe might still be possible (and might even proceed from both ends), the loss of free movement of the sample strand G due to binding at its bases 9750—9800 may reduce the probability of complete displacement, especially when one considers the helical structures actually involved.

Another situation within the scope of the present invention, but also somewhat less preferred than the above, is that in which the labelled polynucleotide binding region (LBR) extends beyond and outside the target binding region (TBR). This second embodiment of the invention is illustrated in Figure 1G (and in Examples 13 and 14), wherein the reagent complex is shown, with the target nucleotide sequence being capable of displacing the labelled polynucleotide as far as the end of the TBR. The labelled polynucleotide would then remain bound via those probe bases which are part of the labelled polynucleotide binding region LBR but not part of the target binding region TBR (such sequence of bases being designated the residual binding region or RBR). Provided that the RBR (e.g., bases 3300 to 3305 of probe P in Figure 1G) is much smaller than the LBR, one could, in theory, change conditions at this point (pH or temperature for example) so as to totally release the labelled polynucleotide L in this complex without releasing other labelled polynucleotides bound to the entire labelled polynucleotide sequence LBR (see Example 13 where this is described for a displacement in solution). One should consider, however, that the hybrid shown in Figure 1G will still be capable of displacement to the left after complete binding of a target nucleotide sequence G, with the labelled polynucleotide capable of displacing part or all of the portion of the target polynucleotide sequence bound to bases within the overlap of TBR and LBR; such an event could be expected to lower the efficiency of displacing the labelled polynucleotide.

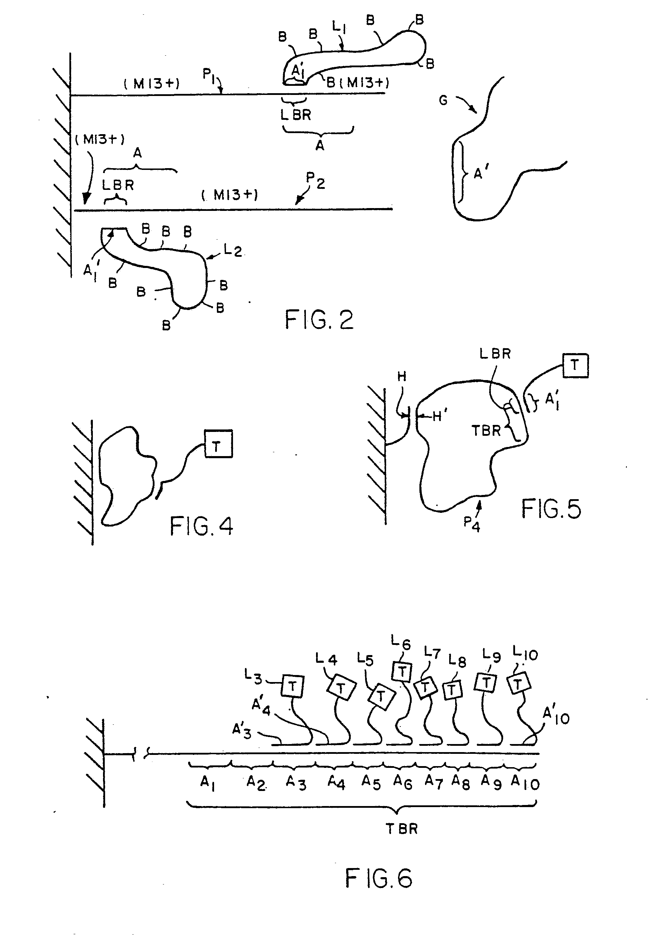

Other embodiments of the invention different from those illustrated in Figures 1A through 1G are illustrated in Figures 2—6 and discussed briefly below.

Figure 2 illustrates a third embodiment of the invention using a complex different from that of Figure 1A in that the labelled polynucleotide $L_1$ is the circular plus (or infectious) strand ($M13^+$) of DNA bacteriophage M13 into which is inserted a segment $A_1'$ complementary to a portion (LBR) of the target binding region A of the probe strands $P_1$ and $P_2$. The label in this embodiment is shown as a series of biotin moieties B distributed within the $M13^+$ major portion of the labelled polynucleotide $L_1$, possibly by growing M13 in bacterial culture with biotinated nucleotide triphosphate (e.g. biotinated—dATP) present or by chemically attaching biotin afterwards to the M13 nucleotides. The $A'_1$ segment may also contain nucleotides bound to biotin. The sample polynucleotide G will, in this instance (Figure 2), contain a target nucleotide sequence A' complementary to segment A of each probe $P_1$ and $P_2$. In this embodiment the probe polynucleotides may also be formed using bacteriophage M13, with segment A complementary to the target nucleotide sequence A' inserted at a location in the non-essential region of the M13 genome. The $M13^+$ segments of the probe are non-complementary (actually equal or homologous) to $M13^+$ regions of the labelled polynucleotide. If the circular phage is cleaved non-specifically (e.g., by partial cleaveage with restriction enzyme Hae III; c.f. R. W. Blakesley and R. D. Wells, *Nature*, vol. 257, pp. 421—422 (1975)), linear strands will be created having segment A positioned at different points relative to the ends, but generally with a portion of the $M13^+$ strand on both sides of segment A. This is illustrated in Figure 2 by strand $P_1$, wherein the segment A is close to the free end of supported polynucleotide, and strand $P_2$, wherein segment A is close to the attached end. Of course, cleaveage will also potentially produce shortened strands (due to two or more cleavages in the $M13^+$ region) and could also produce strands with region A split between the two ends (due to cleavage in region A); the latter condition can result in failure to form a complete and fully operative target binding region. So long as region A is small relative to the length of the $M13^+$ strand (7.2 kilobases; J. Viera & J. Messing, *Gene*, vol. 19, 259—68 (1982)), relatively few probe strands would be expected to have region A split. Uniformity in location of segment A can be achieved by cleaving single-strand M13 DNA specifically (e.g., with a restriction enzyme recognizing an inverted repeat sequence in or inserted into the M13 genome).

After displacement, the biotinated displaced labelled polynucleotides of Figure 2 or related embodiments could be concentrated from the liquid phase, e.g., with a strepavidin column, before readout using, e.g., horseradish peroxidase linked to strepavidin and colorimetric, fluorimetric or chemiluminescent readouts based upon the peroxidase activity.

Figure 3A illustrates a fourth embodiment of the present invention with the reagent complex entirely in solution (no support is present). The probe polynucleotide P has a target binding region (TBR) including a subregion, the labelled polynucleotide binding region (LBR), in which bases are bound to complementary bases of the labelled polynucleotide (L). The labelled polynucleotide (L) contains a fluorescent tag (F) held in the complex in close proximity to a quencher moiety (Q) attached to the probe (P). These moieties F and Q are sufficiently close in the complex of Figure 3A for any signal produced by stimulation of the fluorescent tag (F) by radiation to be absorbed by the quencher moiety (Q). An exemplary pair of F and Q are fluoroscein with rhodamine. See M. Cobb and S. Gotcher, *Am. J. Med. Tech.*, Vol. 48, No. 8, pp. 671—677 (1982).

Figure 3B illustrates the reagent complex of Figure 3A after contact by a sample G containing the base

15

sequence complementary to the entire target binding sequence (TBR). As in Figure 1B, binding will be first to the unpaired region of TBR and then displacing portions of the labelled polynucleotide L from the subregion LBR. The stage of partial displacement (such as seen in Figures 1C and 1D) is illustrated in Figure 3B. Upon completion of the displacement, the target sequence of sample polynucleotide G will be bound to the entire region TBR of probe P and the labelled polynucleotide L will be totally displaced. In this situation, as illustrated in Figure 3C, the tag (F) is now sufficiently far from quencher moiety (Q) to produce a detectable signal on stimulation that is not quenched (except in those rare cases where another probe P happens to be located such that its label Q is in close proximity to tag F). Accordingly, the stimulation of fluorescent tag (F) and measurement of unquenched signals can serve as a quantitative detection method for displaced labelled polynucleotides without a separation step having been performed.

A modification in the embodiment of Figures 3A—3C in which the labelled polynucleotide L and probe polynucleotide P (bearing, respectively, labels F and Q) are part of the same molecule is illustrated in Figure 3D. In addition, a site X is shown which contains an inverted repeat sequence a portion of which is recognizable by a restriction enzyme. Cleavage by such an enzyme can be used to sever the covalent linkage between TBR and L (although this severing is not rqeuired when the read-out is based upon the F—Q interaction). Such cleavage can also be used as a first step in creating a site for attachment of the probe to a support, such attachment to occur before or after displacement. The overall geometry of Figure 3D may thus be used in synthesizing reagent complexes as a single molecule for various embodiments, including those of Figures 1A—1E.

Figure 4 illustrates a complex in which the probe strand $P_3$ is a circular single-stranded polynucleotide non-specifically adsorbed on support S. Such a polynucleotide can be produced by insertion of a target binding region (TBR), into a single-stranded bacteriophage such as M13 through suitable cloning procedures. The labelled polynucleotide L here contains a segment $A'_1$ complementary only to a portion LBR of segment TBR.

Figure 5 illustrates a complex similar to Figure 4 except that, instead of being adsorbed to support S, probe strand $P_4$ is linked via a linking polynucleotide covalently attached to support S. Such linking polynucleotide contains a region H complementary to a segment H' of the probe $P_4$ remote from the TBR segment.

Figure 6 illustrates a complex similar to the embodiment of Figure 1A in that the probe strand $P_5$ contains a target binding region TBR near or at its free end. A number of portions of the TBR segment (subsegments $A_3$, $A_4$, $A_5$, $A_6$, $A_7$, $A_8$, $A_9$, and $A_{10}$) are each base paired to a complementary region of a labelled polynucleotide: segment $A'_3$ of labelled polynucleotide $L_3$ to subsegment $A_3$, etc. Probe $P_5$ contains a target binding region TBR divided into subsegments $A_1$ and $A_2$, together forming the initial binding region, as well as eight additional subsegments ($A_3$ thru $A_{10}$), each forming a labelled polynucleotide binding region. In this embodiment, binding of segment A' of the sample polynucleotide G (as shown in Figure 1E) will displace all of the labelled polynucleotides $L_3$, $L_4$, $L_5$, $L_6$, $L_7$, $L_8$, $L_9$ and $L_{10}$ and thus produce an enhanced signal compared to the situation shown in Figure 1A. It should be appreciated, however, that the absence of any of those labelled polynucleotide (e.g., $L_8$) could permit the target nucleotide to form D-loops (as in Figure 1F) by binding to subsegments $A_1$ and $A_2$ (for which no labelled polynucleotide is complementary) and any other probe subsegment (e.g., $A_8$) for which the labelled polynucleotide is absent on that particular probe strand.

Examples

In certain of the following examples, four small synthetic DNA oligomers were used as the tagged polynucleotides; all were labelled with radioactive phosphorous-32 atoms. Two oligomers were 27 base oligomers matching 26 or 27 bases at the 3' end of one strand of a 1.1 kb pBR322 DNA fragment. This fragment stretched from the Pst I restriction site to the Bam HI restriction site in pBR322 DNA. These two oligomers (L1 and L2), and the labelled polynucleotide binding region to which they were bound, are illustrated in Table 1. The mismatched pyrimidine base in oligomer L2 is underlined.

16

TABLE 1

                                                    350

Probe 5'      end ...     T — A — C — G — C — G — A — T — C — A —

L1                                           3' C — T — A — G — T —

L2                                           3' C — T — A — G — T —

─────────────────────────────────────────────────────────────────

                           360                              367

Probe    T — G — G — C — G — A — C — C — A — C — A — C — C — C —

L1       A — C — C — G — C — T — G — G — T — G — T — G — G — G —

L2       A — C — C — G — C — T — G — G — T — G — T — G — G — <u>C</u> —

              20                                   10    9

─────────────────────────────────────────────────────────────────

              370                              380

Probe    G — T — C — C — T — G — T — G — G — A — T — C — C  ... 3' end

L1       C — A — G — G — A — C — A — C 5' end

L2       C — A — G — G — A — C — A — C 5'  e n d

─────────────────────────────────────────────────────────────────

The two oligomers used in other examples were 32 base oligomers matching a region near the middle of a 1.1 kb human albumin gene sequence. The sequences for the other two oligomers (L3 and L4) are described in Example 7, below.

Example 1

The 27 base oligomer containing a single mismatch (L2 in Table 1) was kinased using $^{32}$P-γ-ATP and hybridized to the + strand of M13mp9 clone II-16 DNA. M13mp9 clone II-16 DNA consists of bacteriophage M13mp9 DNA into which has been closed the 1.1 kb Pst I—Bam HI fragment of pBR322 oriented such that the plus strand contains the Bam HI site at its 3' end. Bound oligomer was then purified on a Sepharose® 4B column to remove unbound oligomer from the reagent complex. Approximately 40% of the probe molecules were found to be hybridized with labelled oligomer. About 250 ng of the reagent complex were exposed to a 5-fold molar excess of single-stranded competitor M13mp8 clone 20 DNA or non-homologous M13mp 8 DNA, at 41°C in the presence of 2× SSC for 20 min or 2 h. The plus strands of M13mp8 clone 20 DNA consists of bacteriophage M13mp8 DNA into which has been cloned the 1.1 kb DNA strand complementary to the pBR322 DNA present in M13mp9 clone II-16. Portions of each sample were removed for gel electrophoresis and autoradiography (data not shown, but they confirmed the subsequent analysis). The remaining samples (5500—8000 cpm total activity) were applied to a 5 ml Sepharose® 4B column. Three drop fractions were eluted in TE (10 mM Tris, pH 8.0, 1 mM Na₂EDTA) and counted with scintillant. The results are summarized in Table 2.

0 167 238

TABLE 2

| Sample | Percent total counts | |
|---|---|---|
| | Early peak (bound oligomer) | Late peak (free oligomer) |
| 1. Original unchallenged reagent complex; 2h, 41°C | 68% | 32% |
| 2. Reagent complex+5-fold molar excess of M13mp8 clone 20 DNA; 20 min, 41°C | 27 | 73 |
| 3. Reagent complex+5-fold molar excess of M13mp8 clone 20 DNA; 2h, 41°C | 5 | 95 |
| 4. Reagent complex+5-fold molar excess of non-homologous M13mp8 DNA; 2h, 41°C | 71 | 29 |

Example 2

Purified reagent comprised of single-stranded M13mp9 clone II-16 DNA (see Example 1) and kinased 27-mer containing a single mismatch (labelled polynucleotide L2 in Table 1, above) were prepared as described in Example 1. Competitive hybridizations were carried out at 50°C in the presence of 2× SSC for up to 1 h; samples were held on ice until subjected to electrophoresis on a 1.5 mm thick 5% (top) and 20% (bottom) stacked polyacrylamide gels. Electrophoresis was conducted for about 600 V-h and the stacked gel system was autoradiographed. Each sample contained 50 ng of the release tag—probe complex; M13mp8 clone 20 DNA served as the sample competitor DNA, while M13mp8 DNA and sheared calf thymus DNA were non-homologous additions. Table 3 summarizes the identity of samples loaded in individual polyacrylamide gel wells and the percent of free or unbound labelled oligomer. The percent oligomer values are derived from densitometric tracings of the autoradiogram taken for each gel lane.

TABLE 3

| Lane | Sample nucleic acid | Time of competitive hybridization | Percent free oligomers |
|---|---|---|---|
| 1. | Orig. hybrid, unchallenged | — | 8 |
| 2. | 250 ng Competitor DNA+1500 ng M13mp8 | 10 min | 87 |
| 3. | 250 ng Competitor DNA+1500 ng M13mp8 | 1 h | 97 |
| 4. | 1500 ng M13mp8 DNA | 1 h | 3 |
| 5. | No competitor DNA | 1 h | 4 |
| 6. | 2000 ng Calf thymus DNA | 1 h | 3 |
| 7. | 250 ng Competitor DNA+2000 ng calf thymus DNA | 10 min | 77 |
| 8. | 250 ng Competitor DNA+2000 ng calf thymus DNA | 1 h | 98 |
| 9. | 250 ng Competitor DNA | 10 min | 69 |
| 10. | 250 ng Competitor DNA | 1 h | 98 |

The percent free oligomer values show that a substantial excess of M13mp8 DNA or complex calf thymus DNA does not cause significant non-specific displacement of the oligomer (labelled polynucleotide) and does not inhibit the rapid displacement caused by competitor DNA (the target nucleotide sequence).

18

Example 3

The rate of displacement of a 27-base oligomer containing a single mismatch (L2, Table 1) was compared to that of a perfectly matched oligomer (L1, Table 1) bound to the same probe polynucleotide sequence. Oligomers were kinased using 32P-γ-ATP to specific activities of $1—2\times10^8$ cpm/µg. Single-stranded probe DNA was prepared by standard procedure from M13mp9 clone II-16 (see Example 1). About 5 µg of the probe polynucleotide were hybridized with 45 ng of either perfect or single-mismatch-containing oligomers (see Table 1) by heating the mixture in the presence of 0.5 M NaCl to 65°C and allowing the solution to cool slowly to room temperature. The hybridized oligomer (reagent complex) was separated from free oligomer by elution from a Sepharose 4B column; approximately 50% of the probe molecules were found to be hybridized to oligomer.

Competition hybridizations were performed at 60°C, in a final volume of 5 µL, containing 2× SSC and 25 ng of the reagent complex. 25 ng of single-stranded M13mp8 clone 20 DNA (see Example 1) served as the competitor DNA having the target nucleotide sequence; non-homologous "competitor" DNA was from bacteriophage M13mp8 viral particles. Samples were placed on ice after incubation for desired periods, and subjected to electrophoresis in a 15% polyacrylamide gel. The area under peaks resulting from densitometric scans of an autoradiogram obtained from the gel were then quantitated. The fraction of total counts which appears as free oligomer (high mobility) and as reagent complex (low mobility) is tabulated below in Table 4 as a function of time of incubation.

TABLE 4

% Total cpm

| Incubation time | Low mobility | | High mobility | |
|---|---|---|---|---|
| | Perfect | Mismatch | Perfect | Mismatch |
| 1. 1× Competitor, 0 min | 100 | — | 0 | — |
| 2. 5 min | 87 | — | 12 | — |
| 3. 15 min | 74 | — | 25 | — |
| 4. 30 min | 60 | 42 | 39 | 58 |
| 5. 1 h | 38 | 37 | 62 | 63 |
| 6. 2 h | 42 | 23 | 57 | 77 |
| 7. 3 h | 42 | 0 | 58 | 100 |
| 8. No competitor, 3 h | 100 | 81 | 0 | 18 |

While 50% of the perfectly matched oligomer is displaced at approximately 40 minutes, about 30 minutes is required to displace specifically 50% of the single mismatch-containing oligomer at the constant reaction temperature of 60°C.

Example 4

An M13 clone containing approximately 1100 bases of an albumin cDNA clone as an insert (representing the target binding region of 1.1 kb in length) was prepared for use as the probe polynucleotide strand. An oligonucleotide 32 bases in length (L3 of Example 7) and complementary to a 32-nucleotide sequence (the labelled polynucleotide binding region) near the middle of the 1.1 kb insert (target binding region) was end-labelled with phosphorous-32 (to be used as the labelled release tag polynucleotide) and hybridized to the M13 probe so as to form a reagent complex. Unhybridized 32-mer was removed by two passages through a 10 cm Sepharose® CL-4B column equilibrated in 0.3M NaCl, 10 mM Tris and 0.1% SDS. The specific activity of the reagent complex was approximately 1000 cpm per ng of probe DNA. Approximately 1/3 of the probe strands had a labelled 32-mer polynucleotide bound. About 10 ng of the reagent complex was then combined in a 20 µl volume of 1 M NaCl, 50 mM Tris with one of the following:

A) 1000 ng sheared, boiled herring sperm (HS) DNA

B) 100 ng boiled, linearized plasmid Alb5/RII DNA containing albumin coding sequence on a 1400 nucleotide fragment (this coding sequence has a portion complementary to the 1.1 kb cloned insert in the above M13 clone and thus represents the target nucleotide sequence);

C) 100 ng of DNA from another M13 clone containing the opposite strand of albumin coding sequence than is present in the probe. This M13 DNA was digested with the restriction endonuclease Hae III to linearize the DNA and to produce a linear fragment (2A/Hae III) containing a 1.72 kb albumin gene sequence which includes a contiguous sequence complementary to the 1.1 kb target binding region sequence).

D) 100 ng of DNA (4A/Hae III) as in C above except that the sequence of DNA coding for albumin that is present is the same as the target binding region in the probe complex.

E) No additional DNA, with incubation.

F) No additional DNA; no incubation.

Each of the above solutions was incubated at 50°C for two hours (except for F). Following incubation, each sample was electrophoresed on a 2.5% agarose gel and the gel was autoradiographed. Several autoradiographs of varying exposure length were prepared. The results with the longest exposure (66 hrs with 2 intensifying screens) indicate that in the absence of true competitor DNA or in the presence of non-homologous competitor, no displacement of the release tag oligonucleotide occurs. However, in the presence of specific competitor, all (in case C) or almost all (in case B) of the release tag oligomer can be displaced. An exposure suitable for densitometry (98 hrs with no screen) was quantified by scanning densitometry. The peaks were carefully cut out of the gel scan, weighed, and the percentage of mass in the intact hybrid (I) and the displaced strand (D) peaks were determined; the results are summarized in the middle columns of Table 5. Following autoradiography, the gel was aligned with the autoradiogram and the regions of the gel containing the radioactivity were excised and counted by liquid scintillation counting. All samples were counted for 20 minutes. The background counts were 25 cpm. This background was subtracted from all samples resulting in the data in the righthand columns of Table 5. The quantification in Table 5 verifies the visual result seen and scored in the autoradiogram; that is, major amounts of displacement occurred only in those runs (cases B and C) in which the complex was contacted with DNA containing a target nucleotide sequence that could bind to the target binding region of the reagent complex.

### TABLE 5
### Quantitated strand displacement in solution

|  |  | Mass (ng) | % | Cpm | % |
|---|---|---|---|---|---|
| A) HS DNA | I | 85.7 | 100 | 245 | 100 |
|  | D | 0 | 0 | 0 | 0 |
| B) Alb 5/RII | I | 25.6 | 32 | 60 | 41 |
|  | D | 54.2 | 68 | 87 | 59 |
| C) 2A/HaeIII | I | 7.1 | 10 | 19 | 24 |
|  | D | 66.0 | 90 | 61 | 76 |
| D) 4A/HaeIII | I | 73.5 | 100 | 250 | 100 |
|  | D | 0 | 0 | (−2) | 0 |
| E) NO DNA-2hr | I | 70.1 | 100 | 218 | 100 |
|  | D | 0 | 0 | 0 | 0 |
| F) NO DNA-0hr | I | 86.4 | 100 | 200 | 100 |
|  | D | 0 | 0 | 0 | 0 |

Example 5

Displacement from a supported complex

The reagent complex described in Example 4 (10 ng) was adsorbed on Schleicher and Schuell NA45 paper (DEAE cellulose/nitrocellulose paper), air dried, and then irradiated with ultraviolet light (handheld short wavelength UV lamp held at a distance of 0.5 cm) for 0, 1, or 5 minutes. The filters were then incubated in a small volume of hybridization solution at 50°C for 4 hours. An aliquot of each solution phase was saved for electrophoresis, and the remaining solution removed and saved. Incubation of all filters was then continued overnight in fresh hybridization solution and aliquots were saved. Displacement reactions were carried out for 4 hours at 50°C with 100 ng of various sample nucleotide strands. An aliquot of each reaction solution phase was again saved for gel electrophoresis and the remainder was counted by liquid scintillation counting for two minutes (no background counting was done). All appropriate samples were electrophoresed on the same 2.5% agarose gel and the gel was autoradiographed. The results indicate that

20

some of the composite reagent complex and some 32-mer came off the filters in the first 4 hours preincubation. The UV treatment for 5 minutes substantially decreased the amount of complex which came off, although a small amount of 32-mer did still come off. During the subsequent overnight incubation, very few additional counts came off according to the results of liquid spectrometry (Table 6). During the displacement reactions, the 32-mer labelled polynucleotide was specifically displaced only when the correct competing DNA strand (containing the 1.1 kb target nucleotide sequence) was present (Table 6, sample competitor 2A/H); and essentially no displacement was detected with either no competitor DNA (No Comp) or a large excess (1000 ng) of non-specific herring sperm competitor DNA (HS DNA).

TABLE 6

| | Competitor DNA | # Cpm in reaction solution phase |
|---|---|---|
| 0′ UV | 2A/H | 1777 |
| | No Comp | 607 |
| | HS DNA | 714 |
| 1′ UV | 2A/H | 2261 |
| | No Comp | 96 |
| | HS DNA | 154 |
| 5′ UV | 2A/H | 2267 |
| | No Comp | 260 |
| | HS DNA | 272 |

Example 6

A portion of pBR322 located between the Eco RI and Sal I restriction sites (about 650 bases in length) was cloned into M13mp8 or M13mp11. Using a $^{32}$P-end labelled 27 nucleotide long oligonucleotide complementary to a portion of this pBR322 sequence as primer and the appropriate template chosen from the above two clones, DNA synthesis was initiated on the template and extended partially around the circle. The DNA was digested with Eco RI and the resulting 375 nucleotide end-labelled fragment was purified by denaturing agarose gel electrophoresis. This 375-mer was annealed back to the pBR322/M13 probe strand to form a reagent complex wherein the labelled polynucleotide was bound to the end 375 nucleotides of a 650 nucleotide target binding region. Unannealed 375-mer was removed by a Sepharose® CL-4$_B$ column as described in Example 4. This reagent complex (10 ng) was then used in a strand displacement reaction at 70°C in 0.4 M NaCl, 100 mM Tris. The sample competitor DNA used in each of the five runs was:

A) no DNA

B) 10 ng of circular M13mp11 into which had been cloned pBR322 DNA complementary to the target binding region over most of its 650 base length; this DNA thus contains the 650 base long target nucleotide sequence in an 7800 base long circle.

C) 100 ng of the same 7800-mer circular DNA as in B;

D) 100 ng of circular M13 into which had been cloned a pBR322 DNA strand identical to that found in the probe strand (and thus does not contain the target nucleotide sequence); and

E) 15 ng of a linearized, denatured 650 base long Eco RI/Sal I fragment of pBR322 (containing the 650 base long target nucleotide sequence as a single-stranded linear DNA molecule as well as its 650 base long complement).

After 0′, 5′, 15′, 30′ and 60′, the displacement reactions were stopped by quenching on ice; and at the end of the experiment, the samples were electrophoresed on a 1.5% agarose gel and autoradiographed. There was a slight contamination with unannealed 375-mer still present (i.e., a background value of radioactivity was seen at the location for 375 nucleotide length pieces in Case A). No increase in this background was observed over the 60′ reaction, either with the negative controls of no competitor DNA (Case A) or with the same strand as sample competitor DNA (Case D). Specific displacement was observed with the opposite strand as competitor (Cases B and C) or with the denatured, linearized fragment (Case E). The ability to quantitate the results of this experiment are complicated by the existence of a doublet of uncertain origin in all cases in the low mobility gel region. Qualitatively, it appeared that the approximately equimolar competitor: reagent ratio of Case B yielded less complete and less rapid displacement compared to the 10:1 molar ratios of Cases C and E (note that the negative control Case D was also at a 10:1 molar ratio). Where displacement occurred in Cases B and C, two new locations of peak radioactivity occurred: one at a lower mobility (higher molecular weight) than the initial complex (presumably an intermediate structure such as in Figure 3B, but with both probe P and competitor G being circular), the other at the higher mobility (lower molecular weight) of displaced 375-mer. The stability of the intermediate structure is presumably due to the circular nature of the probe strand and competitor. Case E does not show the appearance of significant amounts of the lower mobility peak because the challenger is linear in Case E, but does show a second high mobility peak. This second high mobility peak probably results from rehybridization of the displaced labelled 375-mer to the 650 base-long complementary DNA strand present because of the use of denatured double-stranded DNA as sample competitor DNA in Run E.

Example 7

Effect of mismatches on the rate of displacement from and stability of reagent complex

A perfectly matched albumin 32-mer and a 32-mer with 2 mismatches (resulting in G-T mispairing) were end labelled with phosphorus-32 and annealed to an albumin M13 clone as in Example 4. The sequence of the first of these two labelled polynucleotides is:

(Perfectly matched 32-mer): 3'end—CTACAAACGTT—
TTTGATACGACTCCGTTTCCT 5' end (L3).

In the mismatched 32-mer (L4), both underlined deoxycytidines of L3 were replaced by deoxythymidine. The stability of the reagent complexes was determined by melting experiments for 1, 2 and 4 hours. The 32-mer without a mismatch was stable up to 50°C for 2 hours while the 32-mer with two mismatches was stable only up to 40°C for 2 hours.

Each of the reagent complexes was incubated with 100 ng of competing (2A/Hae III) M13-albumin DNA (see Example 4) in a 10 microliter volume for 5, 10, 20, 40, 80, and 120 minutes. The reaction temperatures studied were 40°C for both and also 50°C for the perfect match. The samples were analyzed by electrophoresis and autoradiography as described in Example 4. At 40°C the rate of displacement from the two reagent complexes was nearly identical. However, at 50°C, displacement from the perfectly matched reagent complex was significantly more rapid than from either of the reagent complexes at 40°C. This more rapid displacement is most likely an effect of the higher temperature of hybridization allowable with the perfectly matched reagent complex, the higher temperature resulting in a more optimal rate of hybridization (see J. G. Wetmur and N. Davidson, *J. Mol. Biol.* 31, 349—370 (1968)).

Example 8

Displacement of release tag from nitrocellulose-bound probe by competitor DNA

Single-stranded M13mp9 clone II-16 DNA (see Example 1) was applied in dots (0.25 µg each) to nitrocellulose filters, baked two hours at 80°C and hybridized to a 32p-end labelled 27-mer with a single base mismatch (L2, Table 1) at 50°C in 6× SSC plus 10× Denhardt's solution overnight. After extensive washes in 6× SSC (each dot retaining approximately 25,000 cpm), the dots were placed individually in 1.25 ml reaction mixtures containing 6× SSC plus 10× Denhardt's solution and either no competitor DNA, 2 µg of M13mp8 clone 20 DNA (see Example 1), or 2 µg of M13mp8 DNA (non-homologous competitor. Samples of 100 µl were removed at intervals and counted by liquid scintillation spectrometry (Table 7) reports the results in counts per minutes in the 100 µl aliquots of the supernatant.

TABLE 7

| Displacement time | No competitor DNA | Competitor M13mp8 clone 20 DNA | m13mp8 DNA |
|---|---|---|---|
| 40 min | 40 (0) | 73 (33) | 41 (1) |
| 60 min | 45 (5) | 108 (68) | 44 (4) |
| 125 min | 56 (16) | 195 (155) | 59 (19) |
| 180 min | 73 (33) | 320 (280) | 74 (34) |
| 250 min | 94 (54) | 424 (384) | 92 (52) |
| 395 min | 141 (101) | 652 (612) | 134 (94) |

Values in brackets represent the raw (unbracketed) data after subtracting the 40 cpm machine background. These data show that the sample target nucleotide sequence in M13mp8 clone 20 DNA specifically displaces the radiolabelled release tag oligomer while sample DNA containing no target nucleotide sequence (i.e., M13mp8 DNA) does not displace oligomer above background levels.

Example 9

Use of a reagent complex having a residual binding region

A portion of plasmid pBR322 DNA located between the Eco RI and Sal I recognition sites was cloned into bacteriophage M13mp8 or M13mp11. A 32p-labelled 19 base primer complementary to sequences adjacent to the insert in one of the M13 vectors (designated mp8pBR because M13mp8 was the cloning vector) was hybridized to the insert DNA of that clone and extended *in vitro* with *E. coli* DNA polymerase. The resulting partial double-stranded DNA was digested with the restriction enzyme Bam HI and the 316 base long labelled polynucleotide product was then purified by denaturing agarose gel electrophoresis and hybridized to mp8pBR DNA. The resultant hybrids were purified away from unhybridized labelled polynucleotide by passage over two successive sepharose CL-4B columns.

The competitor for the displacement reaction was the opposite strand of the Eco RI-Sal I fragment cloned in bacteriophage M13mp11 (and therefore designated mp11pBR). This competitor DNA is fully complementary to the 275 bases of the probe which derive from the pBR322 DNA insert in the mp8pBR molecule. An additional 41 base pairs in the labelled polynucleotide are not complementary to the competitoir DNA, since they derive from sequences in the M13 vector. Thus, this probe has a tail (Residual Binding Region) which cannot be displaced by the competitor DNA, but must be melted off after a portion of the labelled polynucleotide has been displaced from the probe.

Displacement of this labelled polynucleotide was shown to occur in two steps, and to require a high reaction temperature. The reagent complex was hybridized to a 10-fold excess of circular or partially digested competitor at 70°C in 100 mM NaCl, 100 mM Tris, pH 8.0 for 2 hours. Aliquots of the reaction mix were then heated for 5 or 15 minutes at either 80°C, 85°C or 90°C. The reactions were then quenched on ice and the samples were analyzed on an agarose gel by electrophoresis followed by autoradiography.

After incubation with circular competitor DNA at 70°C, more than 90% of the reagent complex had become associated with target nucleotide sequence as indicated by a shift in the mobility of the reagent complex on the gel. No such shift was seen when partially digested competitor DNA was used. Because the competitor DNA associates with the probe polynucleotide without displacing the labelled polynucleotide, hybridization of the competitor and reagent complex results in a detectable shift on the autoradiogram of labelled material towards a position of lower mobility. Raising the temperature to within the range of 80°C to 90°C for 5 or 15 minutes resulted in the displacement of labelled polynucleotide from the probe up to a maximum of 80% under the specified conditions.

An experiment similar to the above demonstrated that the observed displacement events were specific. Incubation of the same reagent complex described above with a 10-fold molar excess of nonspecific competitor M13mp7 DNA at 85°C in 100 mM NaCl, 100 mM Tris, pH 7.5 for 2 hours resulted in no displacement above background levels of unbound labelled polynucleotide. The background is likely due to slight contamination of the reagent complex with unhybridized labelled polynucleotide. Incubation of this same reagent complex with an equimolar or 10-fold molar excess of the specific competitor mp11pBR at 85°C under similar conditions again resulted in displacement of up to about 80% of the labelled polynucleotide from the probe.

Example 10
Release tag displacement from a probe polynucleotide tethered on a solid support

Single-stranded DNA from an albumin-M13 clone (4A the probe of Example 4) was prepared and digested with the restriction enzyme Hae III to produce small linear DNA fragments containing human albumin CDNA sequences. The linearized DNA was then tailed with a mixture of nucleotides including a biotinylated derivative of dUTP as follows. The solution for the tailing reaction contained 200 mM potassium cacodylate, 1 mM 2-mercaptoethanol, 1 mM cobalt chloride, 0.05 mM dTTP, 0.05 mM dCTP, and 0.1 mM biotinylated dUTP. Four units of terminal deoxynucleotidyl transferase were added and the reaction mixture incubated for 1 hour at 37°C. Control reactions indicated that several hundred nucleotides were added to each DNA fragment although not all tails contained a biotinylated derivative (presumably due to a slower rate of incorporation of the biotinylated derivative). The tailed DNA was then annealed to 32p-labelled albumin 32-mer L3 as described in Example 4. The annealing reaction mixture (reagent complex) was passed over an 0.4 ml packed volume of avidin-agarose (Sigma) in hybridization buffer (1 M NaCl, 50 mM Tris, pH 8.0, 5× Denhardt's solution). The flow-through was passed over the column again and the column was then washed extensively at room temperature with hybridization buffer. The avidin-agarose beads containing the hybrid were then removed from the column and distributed into 50 μl aliquots in Eppendorf microfuge tubes. There were approximately 30,000 cpm in each aliquot. Various amounts of specific competitor used in Example 4 (between 0 and 200 ng) were added to each microfuge tube and the samples were incubated at 50°C for 2 hours. Following the reaction, the contents of the tubes were transferred into small disposable columns (Bio-RadDispo-columns). Following a brief centrifugation, the eluate solution was recovered in an Eppendorf microfuge tube and quantitated by Cerenkov counting. The eluted cpm were as follows:

TABLE 8

| Amount competitor DNA | Eluted cpm (% of input) |
|---|---|
| 0 | 343 (1.2%) |
| 0.5 | 454 |
| 2 | 553 |
| 10 | 1295 |
| 50 | 4822 |
| 100 | 8965 |
| 200 | 13190 (43%) |

The 0.5 ng sample corresponds to roughly $1 \times 10^8$ competitor DNA molecules detectable (111 cpm above background) in 2 hours. A dose response curve drawn from this experiment shows a high degree of linearity.

Example 11

Hybrids between M13mp9 clone II-16 DNA and the radiolabelled 27 base oligomer L1 were prepared by heating 0.4 pmole M13mp9 clone II-16 DNA plus 2.0 pmole oligomer in 36 μl 5× SSC (1× SSC=0.15 M NaCl, 0.015 M sodium citrate) at 65°C for five minutes, then 50°C for 30 minutes. The solution was fractionated over a 5.0 ml Sepharose® 4B column in 10 mM Tris-HCl, pH 8.1, 1.0 mM Na₂EDTA and radioactivity for each fraction determined by Cerenkov radiation counting. The hybrid (reagent complex) fractions were pooled. The elution profile indicated that more than 45% of the probe strands (M13mp9 clone II-16 DNA) were hybridized to oligomer (1100 cpm per 10 μl). Approximately 10 ng of reagent complex were incubated for 0, 30, 60, 90, 120 or 150 minutes in 20 μl 2× SSC or 5× SSC at 50°C prior to analysis by gel electrophoresis on 15% polyacrylamide slab gels and autoradiography. The results from the autoradiograms showed that there is no spontaneous dissociation of the reagent complex at this temperature ($T_m - 18°$) for at least 150 minutes under conditions which mimic those for competitive strand displacement.

Samples of reagent complex (50 ng) were subsequently mixed with 0% or 8% PEG (MW 6000) in 24 5× SSC and either no competitor DNA or 50 ng of competitor M13mp8 clone 20 DNA and incubated at 50°C for 0, 30, 60 or 90 minutes before analysis by gel electrophoresis in 15% polyacrylamide slab gels. Densitometric scans of the resulting autoradiograms show that strand displacement was barely detectable in the absence of PEG, but significant in the presence of 8% PEG even at the earliest observation time (Table 9). PEG therefore has a significant and specific effect on the rate of strand displacement at this concentration.

**0 167 238**

TABLE 9

| Minutes of incubation | 8% PEG (MW 6000) | 50 ng Competitor DNA | Percent free oligomer |
|---|---|---|---|
| 0 | − | − | 26 |
| | − | + | 21 |
| | + | − | 23 |
| | + | + | 25 |
| 30 | − | + | 28 |
| | − | + | 29 |
| | + | + | 77 |
| | + | + | 79 |
| 60 | − | + | 19 |
| | − | + | 24 |
| | + | + | 94 |
| | + | + | 99 |
| 90 | − | − | 26 |
| | − | + | 33 |
| | + | − | 19 |
| | + | + | 90 |

Example 12

Hybrids bewteen M13mp9 clone II-16 DNA and radiolabelled 27 base oligomer were prepared by incubating 5 μg M13mp9 clone II-16 DNA with 64.5 ng oligomer in 0.5 M NaCl, 10 mM Tris, pH 8.0, 1 mM Na₂EDTA at 65°C for 10 minutes in a waterbath and subsequently allowing the waterbath temperature to slow cool to 30°C. At this time the hybrid DNA was purified over a Sepharose® 4B column. The excluded hybrid DNA fractions were pooled and ethanol precipitated. Hybrid DNA was then resuspended to a concentration of 25 ng/μl in 10 mM Tris, pH 8.0, 1 mM Na₂EDTA. About 25 ng of reagent complex were subsequently incubated at 55°C for 30 minutes or 3 hours in 10 μl of 2× SSC containing 0, 8, 10 or 12 percent polyethylene glycol (PEG), MW6000. All samples were prepared in duplicate with half receiving no addition of competitor DNA and the other half receiving 25 ng competitor M13mp8 clone 20 DNA. Following incubation, the samples were subjected to electrophoresis in 15% polyacrylamide slab gels. The resulting gels were autoradiographed at −80°C with Kodak X-omat® film and Dupont® intensifying screen, and scanned by densitometry.

After 30 minutes incubation at 55°C, the amount of 27 base oligomer DNA displaced from the probe DNA by competitor DNA increased as the percent PEG, MW6000 solution present in the reaction increased from 0 to 12 percent (Table 10). PEG, MW6000 alone did not cause dissociation of the oligomer DNA from the reagent complex in the first thirty minutes in the absence of M13mp8 clone 20 DNA (Table 10). After 3 hours incubation at 55°C, the amount of 27 base oligomer DNA found in the free form was still greater in the presence of PEG, MW6000, at all polymer concentrations than in its absence.

The presence of PEG, MW6000, however, did cause substantial non-specific dissociation of hybridized 27 base oligomer DNA from the reagent complex after 180 minutes at 55°C in the absence of competitor M13mp8 clone 20 DNA (see starred results in Table 10) and therefore conclusions about the specific effect of PEG in promoting strand displacement cannot be drawn for this length period of incubation. In the experiment reported below as Example 15, no such non-specific dissociation was observed in 30, 90, or 150 minutes in the presence of 10% PEG, MW6000. Thereafter, experiments were terminated in under 180 minutes. Example 16, below, shows some non-specific melting (dissociation) of the reagent complex at 60 minutes of incubation under different conditions.

25

TABLE 10

| Minutes of incubation | Percent (w/v) PEG, MW6000 | 25 ng Competitor DNA | Percent free oligomer |
|---|---|---|---|
| 30 | 0 | − | 0 |
| | | + | 0 |
| | 8 | − | 0 |
| | | + | 68 |
| | 10 | − | 0 |
| | | + | 100 |
| | 12 | − | 0 |
| | | + | 100 |
| 180 | 0 | − | 0 |
| | | + | 72 |
| | 8 | − | 47* |
| | | + | 100 |
| | 10 | − | 67* |
| | | + | 100 |
| | 12 | − | 32* |
| | | + | 100 |

The effective weight percent range of PEG, MW6000 on strand displacement reactions was further studied in another experiment. Hybrid DNA was made from $^{32}$P-labelled 27 base oligomer (27-mer) and M13mp9 clone II-16 DNA which had been treated with the restriction enzyme Hinfl. This enzyme cuts the M13mp9 DNA into several linear pieces without interrupting the region in M13mp9 clone II-16 containing the pBR322 DNA insert. The reaction mixture contained 0.8 pmoles of M13mp9 DNA and 6.8 pmoles of 27-mer in 77 µl of 5× SSC. The mixture was fractionated using a Sepharose® CL-4B column connected to a fraction collector and an elution buffer of 10 mM Tris-HCl, pH 8.1, 1.0 mM Na$_2$EDTA. Radioactivity was determined for each fraction by Cerenkov counting. The faster moving peak was pooled as the hybrid. About 45% of the M13mp9 DNA was hybridized to labelled oligomer. About 20 ng of reagent complex in 24 µl of 5× SSC were incubated at 52°C for 90 minutes with 0 or 50 ng of competitor M13mp8 clone 20 DNA in the presence of 0, 5, 12 or 19% PEG, MW6000. The samples were then electrophoresed on a 15% polyacrylamide gel and the resulting gels subjected to autoradiography. Densitometric scans of the autoradiograms show that 5% PEG is ineffective in influencing the extent of strand displacement (Table 11) under these conditions while PEG concentrations of 12% and 19% significantly enhance strand displacement in a specific fashion.

TABLE 11

| Percent (w/v) PEG, MW 6000 | Competitor DNA present | Percent free oligomer |
|---|---|---|
| 0 | − | 38 |
| | + | 47 |
| 5 | − | 37 |
| | + | 47 |
| 12 | − | 42 |
| | + | 87 |
| 19 | − | 37 |
| | + | 100 |

The significant weight percent range of PEG, MW6000 that enhanced strand displacement is thus at least 8—19% under these particular conditions (see 8% in Table 10). Examples below show effects at 2.5% and 5.0% PEG under different buffer conditions.

Example 13

About 25 ng reagent complex DNA were prepared as described in Example 12 and were incubated at 55°C for 60 minutes in 10 µl of 2× SSC containing 10% (w/v) polyethylene glycol of MW 200, 400, 1000, 3350, 6000 or 20,000 daltons. Samples were prepared in duplicate with half of the samples receiving 25 ng competitor M13mp8 clone 20 DNA and half receiving none. Following incubation for 60 minutes, all samples were subjected to electrophoresis in 15% polyacrylamide slab gels. The resulting gels were autoradiographed at −80°C with Kodak® X-omat® film and a Dupont® Cronex® intensifying screen, and the autoradiograms were evaluated by densitometric scanning. Polyethylene glycols of molecular weights 3350, 6000 and 20,000 were more effective in enhancing competitive displacement of hybridized oligomer DNA than the polyethylene glycols of lower molecular weights (Table 12).

TABLE 12

| Polyethylene glycol MW | Percent free oligomer | |
|---|---|---|
| | −Competitor DNA | +Competitor DNA |
| 200 | 0 | 64 |
| 400 | 0 | 60 |
| 1,000 | 0 | 59 |
| 3,350 | 0 | 100 |
| 6,000 | 0 | 100 |
| 20,000 | 0 | 79 |
| Incubated control, no polyethylene glycol | 0 | 24 |

Example 14

Hybrids between M13mp9 clone II-16 DNA and the radiolabelled 27 base oligomer L1 were prepared. About 20 ng of reagent complex in 24 µl of 5× SSC were incubated in the absence or presence of 10% polyethylene glycol (PEG), MW6000 at 52°C for 90 minutes with addition of 0, 2.0, 5.0, 20, 50, or 100 ng of competitor M13mp8 clone 20 DNA. All samples were immediately electrophoresed on 15% polyacrylamide slab gels and the resulting gels subjected to autoradiography. Densitometric scans of the autoradiograms show that the sensitivity of strand displacement reactions is enhanced by the inclusion of 10% PEG, MW6000 (Table 13).

27

TABLE 13

| Inclusion of 10% PEG, MW6000 | Amount of competitor DNA (in ng) | Percent free oligomer |
|---|---|---|
| − | 0 | 38 |
| − | 5.0 | 42 |
| − | 20 | 47 |
| − | 50 | 47 |
| − | 100 | 57 |
| + | 0 | 42 |
| + | 2.0 | 51 |
| + | 5.0 | 83 |
| + | 20 | 93 |
| + | 50 | 87 |
| + | 100 | 90 |

In a related experiment using a more highly purified reagent complex, reagent complex hybrids were prepared and purified as described in Example 12. About 25 ng hybrid DNA were then incubated in the presence of 10 µl 2× SSC and either 2.5, 6.25, 12.5, 25 or 50 ng competitor M13mp8 clone 20 DNA at 55°C for 60 minutes, resulting in ratios of reagent complex DNA:competitor DNA of 1:0.1, 1:0.25, 1:0.5, 1:1.0 or 1:2.0, respectively. The samples were prepared in duplicate with half receiving 10% PEG, MW 6000 and half receiving none. Following incubation, the samples were subjected to electrophoresis on 15% polyacrylamide slab gels and the resulting gels were autoradiographed at −80°C with Kodak X-omat film and a Dupont Cronex intensifying screen. Densitometric scans of the resulting autoradiogram demonstrate that, at all reagent complex DNA:competitor DNA ratios, specific disassociation of hybridized 27 base oligomer from probe DNA by competitor DNA was greater in the presence of 10% PEG, MW6000, than in its absence (Table 14).

TABLE 14

| Amount of reagent complex DNA (in ng) | Amount of competitor DNA (in ng) | % PEG, MW6000 | Percent free oligomer |
|---|---|---|---|
| 25 | 2.5 | 0 | 0 |
| | | 10 | 17 |
| 25 | 6.25 | 0 | 20 |
| | | 10 | 47 |
| 25 | 12.5 | 0 | 13 |
| | | 10 | 83 |
| 25 | 25 | 0 | 32 |
| | | 10 | 100 |
| 25 | 50 | 0 | 41 |
| | | 10 | 100 |

Example 15

Control experiments were carried out to examine non-specific polyethylene glycol (PEG) interactions

28

with reagent complex hybrids for strand displacement. In an initial experiment, hybrids bewteen M13mp9 clone II-16 DNA and the 27 base perfectly matched oligomer L1 were prepared and purified as described in Example 12. About 225 ng hybrid DNA were incubated at 55°C in 90 µl of 2× SSC containing 10% (w/v) PEG, MW 6000. Aliquots of 10 were removed at the start of the experiment and at 30, 90 and 150 minutes. The aliquots were subjected to electrophoresis on 15% polyacrylamide slab gels, the resulting gels were autoradiographed at −80°C and the autoradiograms obtained were evaluated by densitometric scanning using a Shimadzu CS930 plate scanner. The reagent complex was stable as a hybrid DNA under these incubation conditions for up to 2 1/2 hours.

This experiment was terminated at 2.5 hours. In other experiments where mixtures with 10% PEG went for 3 hours (including the experimental values shown by asterisks in Table 10, above), non-specific dissociation of reagent complex hybrids was observed at 3 hours. Rather than attempt to understand this anomalous result, experiments conducted afterwards by the same group were terminated in less than 3 hours.

In a related experiment to study potential aggregation of oligomer and polyethylene glycol (PEG), MW 6000, 0.15 pmole of radiolabelled oligomer was incubated in 20 µl 2× SSC or 5× SSC for 30, 90 or 150 minutes with or without 10% (w/v) PEG 6000 and then analyzed by gel electrophoresis and autoradiography and densitometric scanning. The results demonstrated that oligomer does not spontaneously associate with PEG 6000 and removed the possibility that observed decreases in free oligomer from unpurified reagent complex preparations are artifactual.

Example 16

An 840 base pair Eco RI-Bgl II restriction fragment containing the first 262 codons of the human serum albumin gene was subcloned into the single-stranded phage vector M13mp9, generating the clone designated M13mp9-albumin. (See Lawn et al., *Nucleic Acid Research* vol. 9, pp. 6103—6114 (1981) for the DNA sequence of the human albumin gene). Also inserted into this vector was the Eco RI fragment containing the polylinker from M13mp7. This polylinker sequence, inserted adjacent to the albumin sequence at the Eco RI site, is capable of forming a double-stranded hairpin structure in the otherwise single-stranded molecule. Cleavage of such a molecule with the enzyme Bam HI results in a linear and full length single-stranded molecule. An end-labelled polynucleotide was kinased using $^{32}$P-γ-ATP prepared by hybridizing a 14 base long oligonucleotide (5′ end-GATGCACACAAGAG-3′ end) to sequences within the albumin insert, and extending it *in vitro* using *E. coli* DNA polymerase. The resulting DNA molecules were then digested with Pvu II, which cuts once in the human albumin insert. The final labelled polynucleotide, a 175 base long labelled and single stranded molecule, was then purified by electrophoresis on a denaturing agarose gel. The purified labelled polynucleotide was hybridized to the above M13mp9-albumin strand which serves as the probe polynucleotide; the probe used was either circular or had been linearized with Bam HI. Unhybridized labelled polynucleotide was removed from the reagent complex by passage over two successive Sepharose® CL-4B columns.

Model competitor DNAs used in displacement experiments were either the complementary DNA strand to the human albumin sequence of the M13mp9-albumin clone inserted in an M13mp8 vector (and hereafter designated as M13mp8-albumin), or a denatured double-stranded 840 base pair Eco RI-Bgl II fragment which is colinear with the insert in the M13mp9-albumin clone.

Displacements were carried out by incubating of approximately 10 ng of reagent complex with 100 ng of analyte DNA in 0.2 M NaCl, 100 mM Tris, pH 8.0, at 85°C for 5, 15, 30 and 60 minutes. At appropriate times, samples were placed on ice to stop the reaction, and then all samples were run on a 1.5% agarose gel to assess displacement efficiency.

In the absence of analyte, no melting of the probe-template complex was observed at 5, 15 or 30 minutes, although at 60 minutes approximately 10% of the complex was melted at this temperature. The addition of a tenfold excess of analyte to the displacement reaction resulted in the displacement of approximately 50% of the probe at 15 minutes, 75% at 30 minutes and 95% at 60 minutes. The addition of 2.5% (w/v) PEG, MW 6000 to the reaction mix enhanced the displacement rate two-fold; approximately 95% displacement occurred during 30 minutes incubation. The addition of 5% (w/v) PEG, MW 6000 to the reaction further increased the rate of the displacement reaction significantly: displacement was essentially complete within 15 minutes. The addition of 10% (w/v) PEG, MW 6000 also apparently enhanced the rate of the displacement still further, although the extent of this enhancement could not be accurately estimated from the autoradiograph due to a gel artifact frequently observed with high concentrations of PEG at high temperature in this buffer system. Although this experiment did not include controls of displacement complex without competitor, but in the presence of PEG, another experiment did include such a control. In that experiment, incubation under similar conditions to those of this Example with 10% (w/v) PEG, MW 6000 caused no detectable non-specific melting (dissociation) of the reagent complex in up to 60 minutes of incubation. Thus, increasing concentrations of PEG (at 85°C) appear to specifically increase the rate of the hybridization-strand displacement.

Example 17

A reagent complex was prepared as in Example 4 having a 1.1 kb albumin insert within M13

**0 167 238**

bacteriophage as the target binding region and an end-labelled 32-mer oligomer as the labelled polynucleotide. 10 ng of this reagent complex was combined with either:

a) 100 ng of boiled plasmid pAlb5 digested with Pst I and Bgl II containing an albumin coding sequence complementary to part of the albumin insert in the probe (including the 32 bases bound to radiolabelled 32-mer); or

b) 100 ng of boiled plasmid pUC8 digested with TaqI (this sequence is non-complementary and thus serves as a control).

The samples were combined in a 10 μl volume containing 50 mM Tris, 1M NaCl and either 0, 5, 10, 15 or 20% PEG, MW 6000. Following incubations for 10 minutes at 50°C, the samples were immediately quenched in a dry ice/ethanol bath, thawed on ice and electrophoresed on a 2.5% agarose gel and autoradiographed. The amounts of displaced polynucleotides were estimated visually, as shown in Table 15. The results show no detectable displacement in the absence of the specific analyte at any PEG concentration and a significant enhancement in the amount of displaced labelled polynucleotide in 10 minutes under these conditions in the presence of the specific analyte when 5 or 10% (w/v) PEG, MW 6000 was also present.

TABLE 15

| % PEG, MW 6000 | Non-specific competitor | Specific competitor |
|---|---|---|
| 0 | ND | 10% |
| 5 | ND | 40% |
| 10 | ND | 50% |
| 15 | ND | 20% |
| 20 | ND | 5% |

ND=not detectable, i.e. <2%.

## Claims

1. A method for determining the presence of a predetermined target nucleotide sequence in the nucleic acid of a biological sample which comprises the steps:

(a) providing a reagent complex of (i) a probe polynucleotide which is capable of base pair binding via hyrdogen bonds of purine/pyrimidine bases to the target nucleotide sequence, and (ii) a labelled polynucleotide which is bound by base pair binding via hydrogen bonds of purine/pyrimidine base pairs to the probe polynucleotide in a region of the probe polynucleotide at least partially coextensive with the region in which the probe polynucleotide is capable of binding to the target nucleotide sequence;

(b) contacting the reagent complex with a sample under conditions in which the target nucleotide sequence, if present, binds to the probe polynucleotide and displaces labelled polynucleotide from the complex; and

(c) determining the presence of labelled polynucleotide displaced from the reagent complex.

2. The method of claim 1 wherein the probe polynucleotide contains a target binding region capable of base pair binding to the target nucleotide sequence and a labelled polynucleotide binding region bound to bases of the labelled polynucleotide in the complex, and wherein the labelled polynucleotide binding region is contained within the target binding region.

3. The method of claim 1 or 2 wherein the labelled polynucleotide binding region is about 20 to about 500 nucleotides in length.

4. The method of any previous claim wherein the portion of the target binding region that is not part of the labelled polynucleotide region is at least about 100 nucleotides in length, and is at least as large as the labelled polynucleotide binding region.

5. The method of any previous claim wherein the probe polynucleotide contains a target binding region capable of base pair binding to a sample target nucleotide sequence and contains a labelled polynucleotide binding region bound by purine/pyrimidine base pairing to the labelled polynucleotide of at least about 15 nucleotides; at least some of the nucleotides of the labelled polynucleotide binding region being included in the target binding region; and no more than about 15 nucleotides of the labelled polynucleotide binding region being outside the target binding region.

6. The method of any previous claim wherein the probe polynucleotide is immobilized to a solid support in the complex.

7. The method of claim 6 wherein the determining step (c) comprises:

30

(c1) separating a first phase containing immobilized probe polynucleotide from a second phase comprising displaced labelled polynucleotide; and

(c2) determining the presence of labelled polynucleotide in the second phase.

8. The method of any of claims 1—5 wherein the reagent complex is free in solution during the contacting step (b).

9. The method of claim 8 wherein the determining step (c) comprises:

(c1) separating the reagent complex remaining from a portion of the reaction solution after the contacting step (b), and

(c2) determining the presence of any displaced labelled polynucleotide in a solution phase after separation.

10. The method of any previous claim wherein the base pairing between the labelled polynucleotide and the probe polynucleotide is interrupted by at least one mismatched pair.

11. The method of any previous claim wherein the labelled polynucleotide is a polynucleotide bonded to an enzyme.

12. The method of any previous claim wherein the target nucleotide sequence is DNA.

13. The method of any previous claim wherein the probe polynucleotide is DNA.

14. A method for determining the presence of a predetermined target nucleotide sequence in the nucleic acid of a biological sample which comprises the steps:

(a) providing a reagent complex of (i) a probe polynucleotide which is capable of base pair binding via hydrogen bonds of purine/pyrimidine bases to the target nucleotide sequence, and (ii) a labelled polynucleotide which is bound by base pair binding via hydrogen bonds of purine/pyrimidine base pairs to the probe polynucleotide in a region of the probe polynucleotide at least partially coextensive with the region in which the probe polynucleotide is capable of binding to the target nucleotide sequence;

(b) contacting the reagent complex with a sample under conditions in which the target nucleotide sequence, if present, binds to the probe polynucleotide and displaces labelled polynucleotide from the complex; and

(c) determining the presence of labelled polynucleotide remaining in the reagent complex.

15. The method of any previous claim wherein the conducting step (b) is conducted in the presence of a polyether polymer under conditions in which the target nucleotide sequence, if present, binds to the probe polynucleotide and displaces labelled polynucleotide from the complex, the polyether polymeric agent being of a molecular weight and at a concentration sufficient to increase the rate of appearance of displaced labelled polynucleotide.

16. The method of claim 15 wherein the polyether polymer is a poly(alkylene oxide) of the formula $H(O—R)_n$ wherein R is an alkylene moiety of 1—6 carbons and n is an integer, on a weight average molecular weight basis, of about 35 to about 690.

17. The method of claim 15 wherein the polyether polymer is poly(ethylene oxide).

18. The method of claim 17 wherein the poly(ethylene oxide) is of weight average molecular weight of at least about 1500.

19. The method of any of claims 1—14 wherein the contacting step (b) is conducted in the presence of a volume excluding inert polymeric agent which is non-ionic or anionic of sufficient molecular weight and amount relative to the reagent complex to increase the rate of appearance of displaced labelled polynucleotide.

20. A diagnostic reagent for determining the presence of a predetermined target nucleotide sequence in the nucleic acid of a biological sample comprising:

(i) a probe polynucleotide which is capable of base pair binding via hydrogen bonds of purine/pyrimidine base pairs to the target nucleotide sequence, and

(ii) a labelled polynucleotide which is bound by base pair binding via hydrogen bonds of purine/pyrimidine base pairs to the probe polynucleotide in a region of the probe polynucleotide at least partially coextensive with the region in which the probe polynucleotide is capable of base pair binding to the target nucleotide sequence;

the potential base pair binding between the target nucleotide sequence and the probe polynucleotide being capable of displacing the labelled polynucleotide from the reagent complex.

21. The diagnostic reagent of claim 20 wherein the probe polynucleotide contains a target binding region capable of base pair binding to the target nucleotide sequence and a labelled polynucleotide binding region bound by purine/pyrimidine base pairing to bases of the labelled polynucleotide in the complex, and wherein the labelled polynucleotide binding region is contained within the target binding region.

22. The diagnostic reagent of claim 20 or 21 wherein the labelled polynucleotide binding region is about 20 to about 500 nucleotides in length.

23. The diagnostic reagent of claim 20, 21 or 22 wherein the probe polynucleotide is immobilized to a solid support in the reagent complex.

24. The diagnostic reagent of claim 20, 21 or 22 completely free in solution.

25. The diagnostic reagent of claim 24 wherein the probe polynucleotide contains an attached moiety for affinity separation of reagent complex from displaced labelled polynucleotide.

26. A diagnostic kit for determining the presence of a predetermined target nucleotide sequence in the nucleic acid of a biological sample comprising:

# 0 167 238

(a) the reagent complex of any of claims 20—25, and

(b) a polyether polymer of sufficient molecular weight and amount relative to the reagent complex to increase the rate of appearance of displaced labelled polynucleotide.

27. A diagnostic kit for determining the presence of a predetermined target nucleotide sequence in the nucleic acid of biological sample comprising:

(a) the reagent complex of any of claims 20—25, and

(b) a volume excluding inert polymeric agent which is non-ionic or anionic of sufficient molecular weight and amount relative to the reagent complex to increase the rate of appearance of displaced labelled polynucleotide.

## Patentansprüche

1. Verfahren zur Bestimmung der Gegenwart einer vorbestimmten Zielnukleotidsequenz in der Nukleinsäure einer biologischen Probe mit den Stufen, bei denen man

(a) einen Reagenskomplex von (i) einem Prüfpolynukleotid, das zur Basenpaarbindung über Wasserstoffbindungen von Purin/Pyrimidinbase an die Zielnukleotidsequenz in der Lage ist, und (ii) eines markierten Polynukleotids, das durch Basenpaarbindung über Wasserstoffbindungen von Purin/ Pyrimidinbasenpaaren an das Prüfpolynukleotid in einem Bereich des Prüfpolynukleotids, der sich wenigstens teilweise über den Bereich erstreckt, in welchem das Prüfpolynukleotid sich an die Zielnukleotidsequenz binden kann, gebunden ist, vorsieht,

(b) den Reagenskomplex mit einer Probe unter Bedingungen, bei denen die Zielnukleotidsequenz, wenn vorhanden, sich an das Prüfpolynukleotid bindet und markiertes Polynukleotid aus dem Komplex verdrängt, in Berührung bringt und

(c) das Vorhandensein von markiertem Polynukleotid, das aus dem Reagenskomplex verdrängt wurde, bestimmt.

2. Verfahren nach Anspruch 1, bei dem das Prüfpolynukleotid einen Zielbindungsbereich enthält, der zu einer Basenpaarbindung an die Zielnukleotidsequenz in der Lage ist, und einen Bindungsbereich für markiertes Polynukleotid enthält, der an Basen des markierten Polynukleotids in dem Komplex gebunden ist, und bei dem der Bindungsbereich für markiertes Polynukleotid in dem Zielbindungsbereich enthalten ist.

3. Verfahren nach Anspruch 1 oder 2, bei dem der Bindungsbereich für markiertes Polynukleotid eine Länge von etwa 20 bis etwa 500 Nukleotiden hat.

4. Verfahren nach einem der vorausgehenden Ansprüche, bei dem der Abschnitt des Zielbindungsbereiches, der nicht Teil des markierten Polynukleotidbereiches ist, eine Länge von wenigstens etwa 100 Nukleotiden hat und wenigstens so groß wie der Bindungsbereich für markiertes Polynukleotid ist.

5. Verfahren nach einem der vorausgehenden Ansprüche, bei dem das Prüfpolynukleotid einen Zielbindungsbereich enthält, der zur Basenpaarbindung an eine Proben-Zielnukleotidsequenz in der Lage ist und einen durch Purin/Pyrimidinbasenpaar an das markierte Polynukleotid von wenigstens etwa 15 Nukleotiden gebundenen Bindungsbereich für markiertes Polynukleotid enthält, wobei wenigstens einige der Nukleotide des Bindungsbereichs für markiertes Polynukleotid in dem Zielbindungsbereich enthalten sind und nicht mehr als etwa 15 Nukleotide des Bindungsbereichs für markiertes Polynukleotid außerhalb des Zielbindungsbereichs liegen.

6. Verfahren nach einem der vorausgehenden Ansprüche, bei dem das Prüfpolynukleotid in dem Komplex durch Bindung an einen festen Träger immobilisiert ist.

7. Verfahren nach Anspruch 6, bei dem die Bestimmungsstufe (c) darin besteht, daß man:

(c1) eine erste Phase, die immobilisiertes Prüfpolynukleotid enthält, von einer verdrängtes markiertes Polynukleotid umfassenden zweiten Phase trennt und

(c2) das Vorhandensein von markiertem Polynukleotid in der zweiten Phase bestimmt.

8. Verfahren nach einem der Ansprüche 1 bis 5, bei dem der Reagenskomplex während der Behandlungsstufe (b) frei in Lösung ist.

9. Verfahren nach Anspruch 8, bei dem die Bestimmungsstufe (c) darin besteht, daß man:

(c1) den verbleibenden Reagenskomplex von einem Teil der Reaktionslösung nach der Behandlungsstufe (b) abgetrennt und

(c2) das Vorhandensein von verdrängtem markiertem Polynukleotid in einer Lösungsphase nach der Abtrennung bestimmt.

10. Verfahren nach einem der vorausgehenden Ansprüche, bei dem die Basenpaarbildung zwischen dem markierten Polynukleotid · und dem Prüfpolynukleotid durch wenigstens ein nicht zusammenpassendes Paar unterbrochen wird.

11. Verfahren nach einem der vorausgehenden Ansprüche, bei dem das markierte Polynukleotid ein an ein Enzym gebundenes Polynukleotid ist.

12. Verfahren nach einem der vorausgehenden Ansprüche, bei dem die Zielnukleotidsequenz DNA ist.

13. Verfahren nach einem der vorausgehenden Ansprüche, bei dem das Prüfpolynukleotid DNA ist.

14. Verfahren zur Bestimmung des Vorhandenseins einer vorbestimmten Zielnukleotidsequenz in der Nukleinsäure einer biologischen Probe mit den Stufen, bei denen man

(a) einen Reagenskomplex von (i) einem Prüfpolynukleotid, das zur Basenpaarbindung über Wasserstoffbindungen von Purin/Pyrimidinbasen an die Zielnukleotidsequenz in der Lage ist, und (ii) eines markierten Polynukleotids, das durch Basenpaarbindung über Wasserstoffbindungen von Purin/Pyrimidinbasenpaaren an das Prüfpolynukleotid in einem Bereich des Prüfpolynukleotids, der sich wenigstens teilweise über den Bereich erstreckt, in welchem das Prüfpolynukleotid sich an die Zielnukleotidsequenz binden kann, gebunden ist, vorsieht,

(b) den Reagenskomplex mit einer Probe unter Bedingungen, bei denen sich die Zielnukleotidsequenz, wenn vorhanden, an das Prüfpolynukleotid bindet und markiertes Polynukleotid aus dem Komplex verdrängt, in Berührung bringt und

(c) das Vorhandensein von markiertem Polynukleotid, das in dem Reagenskomplex bleibt, bestimmt.

15. Verfahren nach einem der vorausgehenden Ansprüche, bei dem man die Behandlungsstufe (b) in Gegenwart eines Polyetherpolymers unter Bedingungen, bei denen sich die Zielnukleotidsequenz, wenn vorhanden, an das Prüfpolynukleotid bindet und markiertes Polynukleotid aus dem Komplex verdrängt, durchführt, wobei das polymere Polyethermittel ein ausreichendes Molekulargewicht und eine ausreichende Konzentration hat, um die Geschwindigkeit des Erscheinens von verdrängtem markiertem Polynukleotid zu steigern.

16. Verfahren nach Anspruch 15, bei dem das Polyetherpolymer ein Poly-(alkyelnoxid) der Formel $H(O—R)_n$ ist, worin R ein Alkylenrest mit 1 bis 6 Kohlenstoffatomen ist und n eine ganze Zahl, auf Molekulargewichtsgrundlage (Gewichtsmittel), von etwa 35 bis etwa 690 ist.

17. Verfahren nach Anspruch 15, bei dem das Polyetherpolymer Poly-(ethylenoxid) ist.

18. Verfahren nach Anspruch 17, bei dem das Poly-(ethylenoxid) ein Molekulargewicht (Gewichtsmittel) von wenigstens etwa 1500 hat.

19. Verfahren nach einem der Ansprüche 1 bis 14, bei dem man die Behandlungsstufe (b) in Gegenwart eines volumenausschließenden inerten polymeren Mittels durchführt, das nichtionisch oder anionisch ist und ausreichendes Molekulargewicht hat und in einer solchen Menge in Bezug auf den Reagenskomplex vorliegt, daß es die Geschwindigkeit des Erscheinens von verdrängtem markiertem Polynukleotid steigert.

20. Diagnostisches Reagens zur Bestimmung des Vorhandenseins einer vorbestimmten Zielnukleotidsequenz in der Nukleinsäure einer biologischen Probe mit

(i) einem Prüfpolynukleotid, das zur Basenpaarbindung über Wasserstoffbindungen von Purin/Pyrimidinbasenpaaren an die Zielnukleotidsequenz in der Lage ist, und

(ii) einem markierten Polynukleotid, das durch Basenpaarbindung über Wasserstoffbindungen von Purin/Pyrimidinbasenpaaren an das Prüfpolynukleotid in einem Bereich des Prüfpolynukleotids, der sich wenigstens teilweise über den Bereich erstreckt, in welchem das Prüfpolynukleotid zur Basenpaarbindung an die Zielnukleotidsequenz in der Lage ist, gebunden ist, wobei die potentielle Basenpaarbindung zwischen der Zielnukleotidsequenz und dem Prüfpolynukleotid das markierte Polynukleotid aus dem Reagenskomplex verdrängen kann.

21. Diagnostisches Reagens nach Anspruch 20, bei dem das Prüfpolynukleotid einen Zielbindungsbereich enthält, der zur Basenpaarbindung an die Zielnukleotidsequenz in der Lage ist, und einen Bindungsbereich für markiertes Polynukleotid enthält, der durch Purin/Pyrimidinbasenpaarbildung an Basen des markierten Polynukleotids in dem Komplex gebunden ist, und wobei der Bindungsbereich für markiertes Polynukleotid in dem Zielbindungsbereich enthalten ist.

22. Diagnostisches Reagens nach Anspruch 20 oder 21, bei dem der Bindungsbereich für markiertes Polynukleotid eine Länge von etwa 20 bis etwa 500 Nukleotiden hat.

23. Diagnostisches Reagens nach Anspruch 20, 21 oder 22, bei dem das Prüfpolynukleotid durch Bindung an einen festen Träger in dem Reagenskomplex immobilisiert ist.

24. Diagnostisches Reagens nach Anspruch 20, 21 oder 22, vollständig frei in Lösung.

25. Diagnostisches Reagens nach Anspruch 24, bei dem das Prüfpolynukleotid einen angefügten Rest für Affinitätstrennung des Reagentskomplexes von verdrängtem markiertem Polynukleotid enthält.

26. Diagnostische Anordnung zur Bestimmung des Vorhandenseins einer vorbestimmten Zielnukleotidsequenz in der Nukleinsäure einer biologischen Probe mit

(a) dem Reagenskomplex nach einem der Ansprüche 20 bis 25 und

(b) einem Polyetherpolymer mit ausreichendem Molekulargewicht und ausreichender Menge in Bezug auf den Reagenskomplex, um die Geschwindigkeit des Erscheinens von verdrängtem markiertem Polynukleotid zu steigern.

27. Diagnostische Anordnung zur Bestimmung des Vorhandenseins einer vorbestimmten Zielnukleotidsequenz in der Nukleinsäure einer biologischen Probe mit

(a) dem Reagenskomplex nach einem der Ansprüche 20 bis 25 und

(b) einem volumenausschließenden inerten polymeren Mittel, das nicht-ionisch oder anionisch ist und ein genügendes Molekulargewicht hat und in ausreichender Menge in Bezug auf den Reagenskomplex vorliegt, um die Geschwindigkeit des Erscheinens von verdrängtem markiertem Polynukleotid zu steigern.

**Revendications**

1. Un procédé pour la détermination de la présence d'une séquence cible prédéterminée de nucléotides dans l'acide nucléique d'un échantillon biologique qui comprend les stades consistant à:

# 0 167 238

(a) se pourvoir d'un complexe réactif de (i) un polynucléotide sonde capable de liaison de paires de bases par l'intermédiaire de liaisons hydrogène de bases puriques/pyrimidiques avec la séquence cible de nucléotides et (ii) un polynucléotide marqué qui est lié par liaison de paires de bases par l'intermédiaire de liaisons hydrogène de paires de bases puriques/pyrimidiques au polynucléotide sonde dans une région du polynucléotide sonde s'étendant au moins partiellement dans la région où le polynucléotide sonde est capable de liaison avec la séquence cible de nucléotides;

(b) mettre le complex réactif au contact d'un échantillon dans des conditions telles que la séquence cible de nucléotides, lorsqu'elle est présente, se lie au polynucléotide sonde et déplace le polynucléotide marqué du complexe; et

(c) déterminer la présence du polynucléotide marqué déplacé du complexe réactif.

2. Le procédé de la revendication 1 dans lequel le polynucléotide sonde contient une région de liaison de la cible capable de liaison de paires de bases avec la séquence cible de nucléotides et une région de liaison de polynucléotide marqué liée aux bases du polynucléotide marqué dans le complexe, et dans lequel la région de liaison du polynucléotide marqué est contenue dans la région de liaison de la cible.

3. Le procédé de la revendication 1 ou 2 dans lequel la région de liaison du polynucléotide marqué a une longueur d'environ 20 à environ 500 nucléotides.

4. Le procédé de l'une quelconque des revendications précédentes dans lequel la portion de la région de liaison de la cible, qui ne fait pas partie de la région de liaison du polynucléotide marqué, a une longueur d'au moins environ 100 nucléotides et est au moins aussi importante que la région de liaison du polynucléotide marqué.

5. Le procédé de l'une quelconque des revendications précédentes dans lequel le polynucléotide sonde contient une région de liaison de la cible capable de liaison de paires de bases avec une séquence cible de nucléotides de l'échantillon et contient une région de liaison du polynucléotide marqué liée par appariement de bases puriques/pyrimidiques avec le polynucléotide marqué d'au moins environ 15 nucléotides; au moins une partie des nucléotides de la région de liaison de polynucléotide marqué étant incluse dans la région de liaison de la cible; et pas plus d'environ 15 nucléotides de la région de liaison du polynucléotide marqué étant à l'extérieur de la région de liaison de la cible.

6. Le procédé de l'une quelconque des revendications précédentes dans lequel le polynucléotide sonde est immobilisé sur un support solide dans le complexe.

7. Le procédé de la revendication 6 dans lequel le stade de détermination (c) comprend:

(c1) la séparation d'une première phase contenant le polynucléotide sonde immobilisé d'avec une seconde phase comprenant le polynucléotide marqué déplacé; et

(c2) la détermination de la présence du polynucléotide marqué dans la seconde phase.

8. Le procédé de l'une quelconque des revendications 1 à 5, dans lequel le complexe réactif est libre en solution pendant le stade de contact (b).

9. Le procédé de la revendication 8 dans lequel le stade de détermination (c) comprend:

(c1) la séparation du complexe réactif restant d'avec une portion de la solution réactionnelle après le stade de contact (b), et

(c2) la détermination de la présence de tout nucléotide marqué déplacé dans une phase de solution après la séparation.

10. Le procédé de l'une quelconque des revendications précédentes dans lequel l'appariement de bases entre le polynucléotide marqué et le polynucléotide sonde est interrompu par au moins une paire présentant un défaut d'appariement.

11. Le procédé de l'une quelconque des revendications précédentes dans lequel le polynucléotide marqué est un polynucléotide lié à une enzyme.

12. Le procédé de l'une quelconque des revendications précédentes dans lequel la séquence cible de nucléotides est constituée d'ADN.

13. Le procédé de l'une quelconque des revendications précédentes dans lequel le polynucléotide sonde est constitué d'ADN.

14. Un procédé pour la détermination de la présence d'une séquence cible prédéterminée de nucléotides dans l'acide nucléique d'un échantillon biologique, qui comprend les stades consistant à:

(a) se pourvoir d'un complexe réactif de (i) un polynucléotide sonde capable de liaison de paires de bases par l'intermédiaire de liaisons hydrogène de bases puriques/pyrimidiques avec la séquence cible de nucléotides et (ii) un polynucléotide marqué qui est lié par liaison de paires de bases par l'intermédiaire de liaisons hydrogène de paires de bases puriques/pyrimidiques au polynucléotide sonde dans une région du polynucléotide sonde s'étendant au moins partiellement dans la région où le polynucléotide sonde est capable de liaison avec la séquence cible de nucléotides;

(b) mettre le complexe réactif au contact d'un échantillon dans des conditions telles que la séquence cible de nucléotides, lorsqu'elle est présente, se lie au polynucléotide sonde et déplace le polynucléotide marqué du complexe; et

(c) déterminer la présence du polynucléotide marqué demeurant dans le complexe réactif.

15. Le procédé de l'une quelconque des revendications précédentes dans lequel la réalisation du stade (b) est effectuée en présence d'un polymère polyéther dans des conditions telles que la séquence cible de nucléotides, lorsqu'elle est présente, se lie au polynucléotide sonde et déplace le polynucléotide marqué

34

du complexe, l'agent polyéther polymère ayant un poids moléculaire et une concentration suffisants pour accroître le taux d'apparition du polynucléotide marqué déplacé.

16. Le procédé de la revendication 15 dans lequel le polymère polyéther est un poly(oxyde d'alcoylène) de formule $H(O-R)_n$ dans laquelle R est un fragment alcoylène de 1 à 6 carbones et n est un entier d'environ 35 à environ 690 en base moyenne pondérale du poids moléculaire.

17. Le procédé de la revendication 15 dans lequel le polymère polyéther est un poly(oxyde d'éthylène).

18. Le procédé de la revendication 17 dans lequel le poly(oxyde d'éthylène) a une moyenne pondérale du poids moléculaire d'au moins environ 1 500.

19. Le procédé de l'une quelconque des revendications 1 à 14 dans lequel le stade de contact (b) est effectué en présence d'un agent polymère inerte d'exclusion de volume qui est non-ionique ou anionique dont le poids moléculaire et la quantité relative au complexe réactif sont suffisants pour accroître le taux d'apparition du polynucléotide marqué déplacé.

20. Un réactif de diagnostic pour la détermination de la présence d'une séquence cible prédéterminée de nucléotides dans l'acide nucléique d'un échantillon biologique, comprenant:

(i) un polynucléotide sonde qui est capable de liaison de paires de bases par l'intermédiaire de liaisons hydrogène de paires de bases puriques/pyrimidiques avec la séquence cible de nucléotides, et

(ii) un polynucléotide marqué qui est lié par liaison de paires de bases par l'intermédiaire de liaisons hydrogène de paires de bases puriques/pyrimidiques au polynucléotide sonde dans une région du polynucléotide sonde s'étendant au moins partiellement dans la région où le polynucléotide sonde est capable de liaison de paires de bases avec la séquence cible de nucléotides;

la liaison de paires de bases potentielle entre la séquence cible de nucléotides et le polynucléotide sonde étant capable de déplacer le polynucléotide marqué du complexe réactif.

21. Le réactif de diagnostic de la revendication 20 dans lequel le polynucléotide sonde contient une région de liaison de la cible capable de liaison de paires de bases avec la séquence cible de nucléotides et une région de liaison du polynucléotide marqué liée par appariement de bases puriques/pyrimidiques aux bases du polynucléotide marqué dans le complexe et dans lequel la région de liaison du polynucléotide marqué est contenue dans la région de liaison de la cible.

22. Le réactif de diagnostic de la revendication 20 ou 21 dans lequel la région de liaison du polynucléotide marqué a une longueur d'environ 20 à environ 500 nucléotides.

23. Le réactif de diagnostic de la revendication 20, 21 ou 22 dans lequel le polynucléotide sonde est immobilisé sur un support solide dans le complexe réactif.

24. Le réactif de diagnostic de la revendication 20, 21 ou 22 complètement libre en solution.

25. Le réactif de diagnostic de la revendication 24 dans lequel le polynucléotide sonde contient un fragment fixé pour la séparation par affinité du complexe réactif d'avec le polynucléotide marqué déplacé.

26. Une trousse diagnostique pour la détermination de la présence d'une séquence cible prédéterminée de nucléotides dans l'acide nucléique d'un échantillon biologique comprenant:

(a) le complexe réactif de l'une quelconque des revendications 20 à 25, et

(b) un polymère polyéther dont le podis moléculaire et la quantité relative au complexe réactif sont suffisants pour accroître le taux d'apparition du polynucléotide marqué déplacé.

27. Une trousse diagnostique pour la détermination de la présence d'une séquence cible prédéterminée de nucléotides dans l'acide nucléique d'un échantillon biologique comprenant:

(a) le complexe réactif de l'une quelconque des revendications 20 à 25, et

(b) un agent polymère inert d'exclusion de volume qui est non-ionique ou anionique dont le poids moléculaire et la quantité relative au complexe réactif sont suffisants pour accroître le taux d'apparition du polynucléotide marqué déplacé.

FIG. IA

FIG. IB

FIG. IC

FIG. ID

FIG.IE

FIG.IF

FIG.IG

FIG. 2

FIG. 4

FIG. 5

FIG. 6

FIG.3A

FIG.3B

FIG.3C

FIG.3D